# EUROPEAN PATENT APPLICATION

(11) **EP 4 685 151 A1**
(43) Date of publication of application: **28.01.2026**
(21) Application number: 24382811.8
(22) Date of filing: 24.07.2024
(51) Int. Cl.: C07K 5/087, C07K 5/107, A61P 35/00

(54) **PROTEASOME INHIBITOR**

(71) Applicant: Max-Planck-Gesellschaft zur Förderung der Wissenschaften e.V., 80539 München (DE); Universitat Jaume I, 12071 Castellón De La Plana (ES)
(72) Inventor: Chari, Ashwin, 37070 Goettingen (DE); Henneberg, Fabian, 37070 Goettingen (DE); González Adelantado, Florenci Vicent, 12071 Castellón de la plana (ES); Bou Iserte, Lledó, 12071 Castellón de la plana (ES)
(74) Representative: Pons IP

(57) **Abstract**

Proteasome inhibitors of formula I, wherein the meanings for the various substituents are as disclosed in the description. 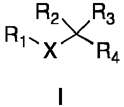 The warhead is extended with a moiety facilitating pi-pi-interaction with Tyr169

## Description

The present invention relates to a new series of proteasome inhibitors of formula I as well as to a pharmaceutical compositions comprising them and to their use as a medicament.

### BACKGROUND ART

Proteasomes represent attractive targets for the search of antineoplastic drugs and immunomodulating agents. Proteasomes function as waste managers in cells to hydrolyze and eliminate unneeded or damaged proteins. Inhibition of the proteasome causes cancer cell apoptosis (death) due to the buildup of waste proteins. Velcade^{®} (Bortezomib) is the first-in-class proteasome inhibitor as a commercial drug currently employed for the treatment of patients with multiple myeloma and mantle cell lymphoma (US8962572B2). It is a modified dipeptidyl boronic acid which reversibly inhibits the protease activity of the 26S proteasome (Structure 2006, 14, 451-56).

Then commercial drug Kyprolis^{®} (carfilzomib) was developed starting from epoxyketone natural product epoxomicin. It is indicated in combination with dexamethasone or with lenalidomide plus dexamethasone for the treatment of patients with relapsed or refractory multiple myeloma who have received one to three lines of therapy. A total of 220 international patents relates to KYPROLISO (US7491704, US8129346, US8207127, US8207125, US8207042, US8207126, US7232818, US8207297, US7737112, US9493582, US9511109, USRE47954) although the manufacturing process for the epoxide remains uncertain based on public disclosed information (OPR&D 2016, 20, 2028-2042). It is marketed in Europe (EU/1/15/1060) by Amgen Europe. Carfilzomib is a tetrapeptide with an asymmetric epoxide that covalently reacts with N-terminal threonines in the active site of the 20S proteasome, resulting in irreversible inhibition of the proteasome and death of cancer cells.

An oral form of peptide boronic acid as bortezomib is Ninlaro^{®} (ixazomib) employed as a drug for the treatment of multiple myeloma (WO2017174046).

Although Velcade^{®}, Kyprolis^{®} and Ninlaro^{®} are currently used they are not curative, and overcoming proteasome inhibitors resistance to extend patient survival remains a major unmet need (Trends in Pharmacological Sciences 2023, 44, 8, 507-518). In that sense, new proteasome inhibitors specific for any of the catalytic subunits (β1, β2, and β5) might afford alternative chemotherapies to circumvent the resistance associated to the use of current inhibitors. Immunoproteasome, an alternative form of proteasome, has been described to include different proteolytic beta subunits (β1i, β2i, and β5i subunits instead of β1, β2, and β5 subunits) in the 20S core (Adv. Exp. Med. Biol. 2020; 1243: 147-162).

Besides the commercial drugs, a previous patentability report of this work made by the European Patent Office found other reported (patent or non-patent documents) proteasome inhibitors (Biomolecules, 2022, 12, 54; J. Med. Chem. 2019, 62, 1626-1642; J. Med. Chem. 2016, 59, 7177-7187; J. Am. Chem. Soc. 2016, 138, 9874-9880). They are beta-5c inhibitors (ONX-0914, KZR-616, LU-005i, PR-924, KZR-329 and LU015i) or beta-5i inhibitors (CPSI(PR-893), trans-LU-oo5c, trans-LU-015c, PR-825, compound 4h), beta-1c/beta-1i (HO-282, NC-001, NC-021, YU-102, compound 5 and az-NC-001), beta-1i (DB-310, KZR-504, LU-001i, DB-60, ML604440, UK101, LU-001c, compound 10). The peptidyl reported compounds are all of them modified peptides having an epoxyketone moiety as a warhead as epoxomicin or carfilzomib or boronic acid as bortezomib (ML604440). For the beta-2 inhibitors they are epoxyketones (LU-002i, compound 87, compound 10), or vinyl sulfones (LU-102, LU002c and LU-012c). For beta-2i/beta-5i, compound 39 is also an epoxyketone, and glidobactins are enones active as beta-2/beta-5.

The inhibitory mode of action of epoxyketone inhibitors, such as epoxomicin, carfilzomib or dihydroeponemycin has been described based on several crystal structures with human 20S proteasome. The gamma-hydroxyl group of the catalytic threonine Thr-1 reacts with the ketone group, and the amino group of the Thr-1 reacts with the terminal carbon atom of the epoxide. This results in the formation of an oxazepane ring product, as shown in Scheme 1 (Schrader et al., Science, 353, 594-598 (2016)).

Accordingly, it would be desirable to provide novel compounds that are capable of inhibiting constitutive proteasome and/or immunoproteasome, and additionally specific inhibitors of the catalytic subunits of proteasome. Compounds should exhibit good activity in *in vivo* pharmacological assays, good oral absorption when administered by the oral route, as well as be metabolically stable and exhibit a favorable pharmacokinetic profile. Moreover, compounds should not be toxic and exhibit few side effects.

### SUMMARY OF THE INVENTION

A first aspect of the present invention related to a compound of formula **I**: 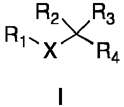 or a pharmaceutically acceptable salt thereof, wherein:
X is C=O or C=S or CH-OCOCH₃;
R₁ is a peptidic group binding to a proteolytic site of a proteasome, wherein said peptidic group is bound to X, comprises at least two amino acids, and X takes the place of the carbonyl group of the C-terminal amino acid; and optionally a second group enhancing delivery, wherein said second group is bound to the N-terminus of said peptidic group;
when R₂ is CH₂ or CH-alkyl, R₃ is O, whereby R₂ and R₃ forming an epoxide ring; or when R₂ is CH=O, CH₂-I, CH₂-Br, CH₂-Cl, CH₂-OPO(OH)₂, CH₂-p-toluene sulfonyloxy (CH₂-OTs), CH₂-methane sulfonyloxy (CH₂-Oms) or CO- N-oxy-succinimide (CO-NHS), R₃ is H or methyl or CF₃;
R₄ is C(O)-YR₅ or C(S)-YR₅, or CH₂YR₅;
Y is NH, C, O or S; and
R₅ is a group occupying the new site binding, particularly an alkyl, aryl or heteroaryl group to establish pi-pi interactions with Tyr-169, that group may be substituted, allowing formation of hydrogen bonds with Thr-21 (constitutive proteasome) / Ser-21 (immunoproteasome), an aromatic ring present in an amino acid containing an aromatic group or R₅ is a peptidic group.

Some compounds of formula I can have chiral centers that can give rise to various stereoisomers. The present invention relates to each of these stereoisomers and also mixtures thereof. Among all stereoisomers, the preferred configuration of the stereocenter of C-terminal amino acid is S, and the stereogenic carbon bound to X, R2, R3 and R4 of formula I would be R for inhibitors selective towards beta-5c subunit, and S for beta-1i selectivity.

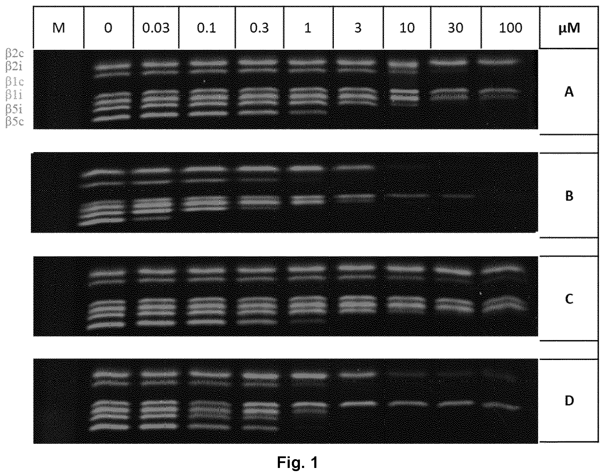

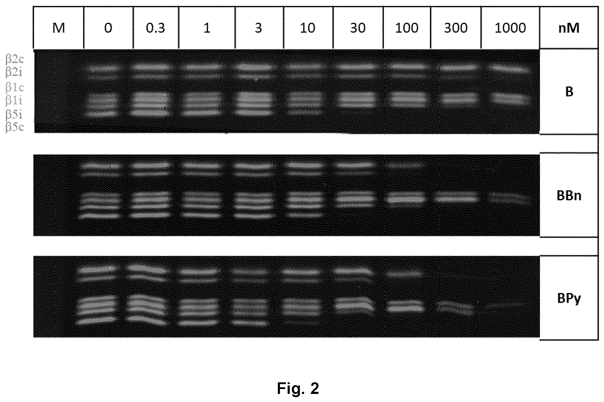

Compounds A, B, C, D, BBn and Bpy were assayed against three catalytic subunits beta-1, beta-2 and beta-5 of constitutive and immune proteasome using selective fluorescent probes (Fig.1 and Fig.2). All compounds were active in the nanomolar range with Bpy being most active one.

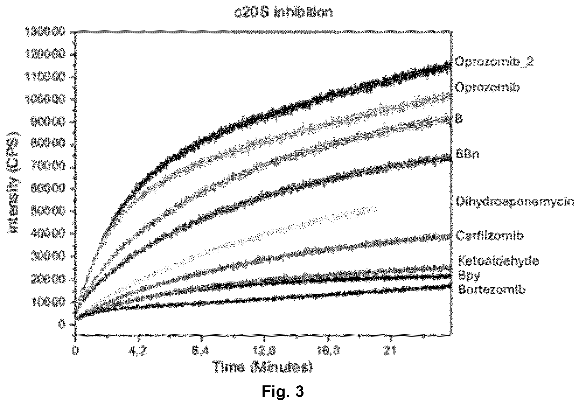

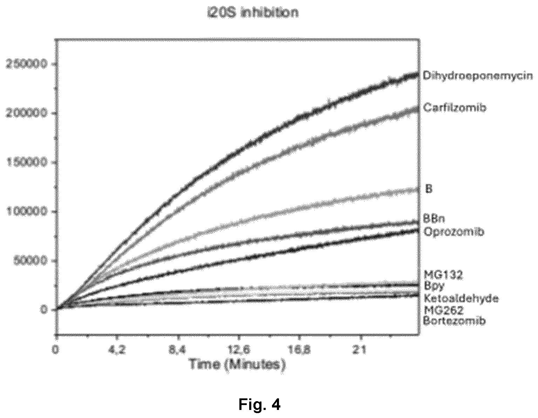

Fluorescence progress curves against c20s and i20s proteasome for most active inhibitors B, BBn and BPy, were performed and compared to already known proteasome inhibitors (oprozomib, dihydroeponemycin, carfilzomib, ketoaldehyde and bortezomib) (Fig.3 and Fig.4). It shows compounds B, BBn and Bpy compounds shown similar activity to already known proteasome inhibitors.

A number of 10 crystal structures of the human constitutive 20S and immuno-20S proteasome at improved resolutions ranging from 2.0 - 2.5Å. These new crystal structures of human 20S proteasomes provide atomic models of the inhibited state. The formation of a 7-membered 1,4-oxazepane ring structure is visualized involving the catalytic threonine residue of the proteasome active site and the inhibitor BPy in the inhibited state (Fig.5, Fig.6 complex BPy-c20S) (Fig.7, Fig.8 complex BPy-i20S). Formation of such an 1,4-oxazepane inhibited state is achieved by a double electrophilic reactive sites on the inhibitor (carbonyl and epoxide). In the crystal structures, it is clearly observed the pending aromatic ring of the inhibitor (pyridine for BPy) occupying a new site in an adequate distance and orientation to pi-pi interact with the phenolic aromatic ring of Tyrosine-169 (Scheme 1).

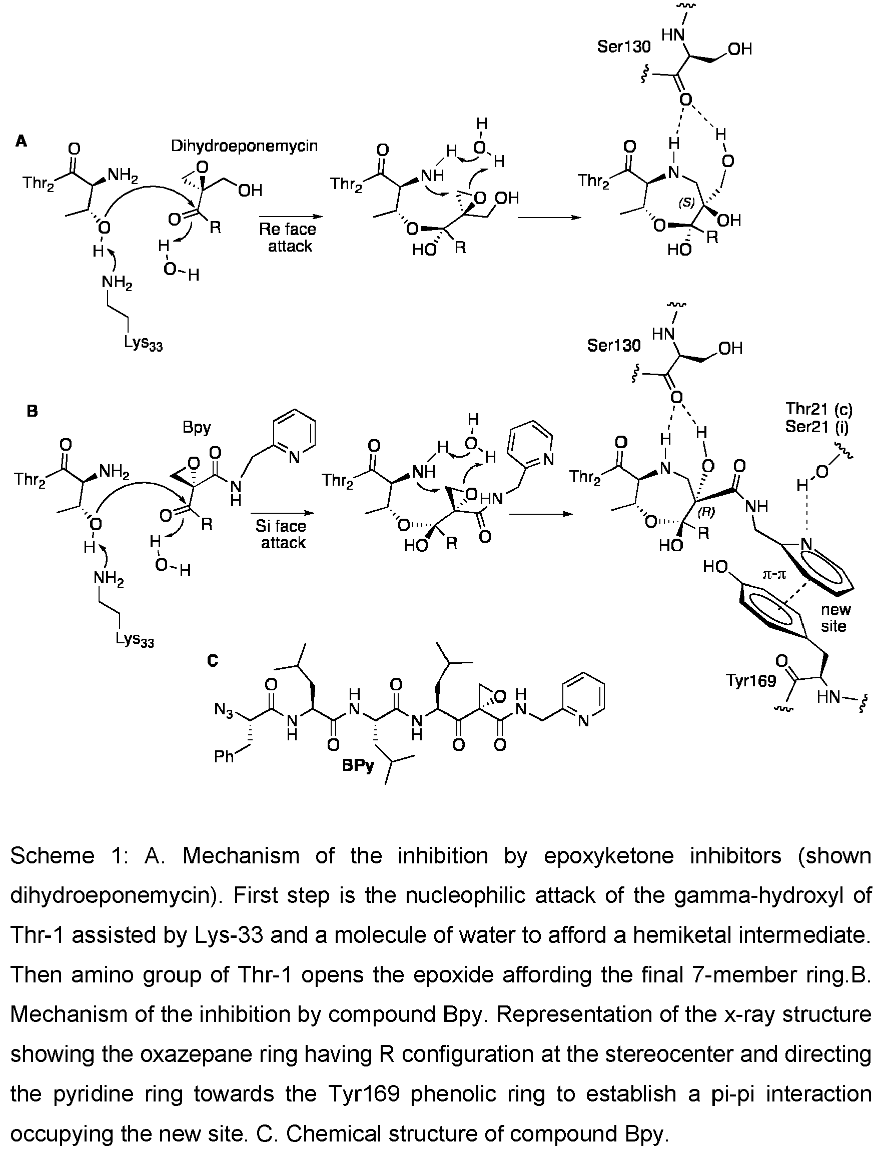

The occupancy of this site and the interaction between inhibitors and catalytic subunits of proteasome is not observed in the previously reported epoxyketone inhibitors. The occupancy of the new site can be established as a consequence of the Si face attack of the hydroxyl group of Thr-1 to the ketone, the stereochemistry of the epoxide (in contrast to the previously known epoxyketone inhibitors epoxomicin, dihydroeponemycin, oprozomib, carfilzomib,... where the 1,4-oxazepane 7-ring is established by a Re face attack of the hydroxyl group of Thr-1 to the ketone) and the presence of a pyridine ring to occupy the new site through pi-pi interaction (Thr-169) and hydrogen bonding with Thr-21 (constitutive proteasome) or Ser-21 (immune proteasome) (Scheme 1). The second attack of the amine of Thr-1 to the epoxide gives rise to an oxazepane with R configuration on the tertiary alcohol giving rise to the pyridine ring occupying the new site. The knowledge of the mechanism allows for a certain site to be exploited and therefore to formulate the compounds in this application. 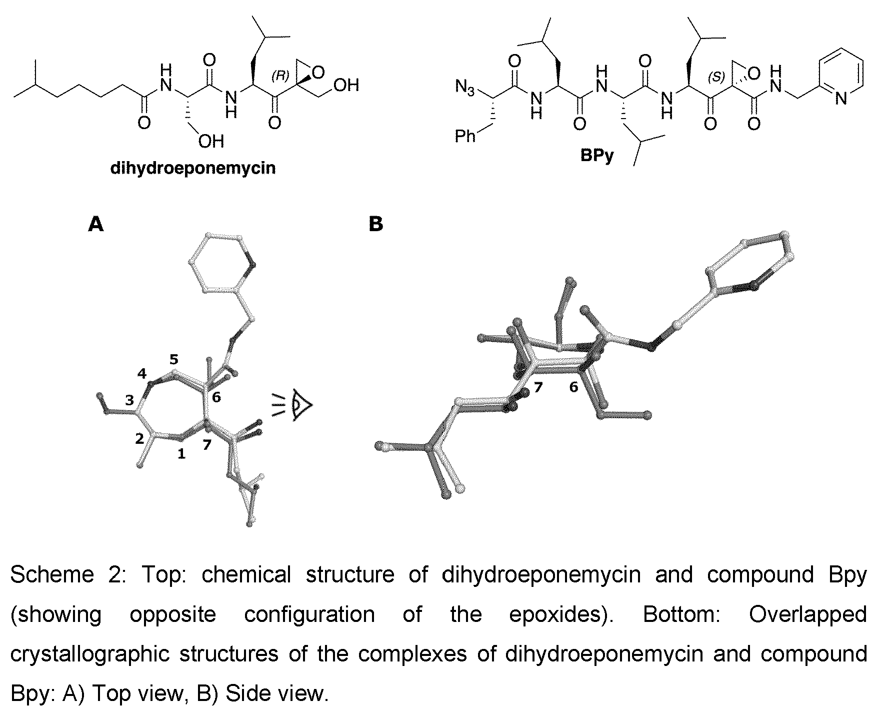 Scheme 2: Top: chemical structure of dihydroeponemycin and compound Bpy (showing opposite configuration of the epoxides). Bottom: Overlapped crystallographic structures of the complexes of dihydroeponemycin and compound Bpy: A) Top view, B) Side view.

Although peptidic compounds are considered, the present invention would include any chemical group targeting proteolytic site in the proteasome or to the vicinity is a suitable R₁ group. The present invention extends to non-peptidic proteasome inhibitors occupying S1-S3 sites of the active sites, R₁ group of the formula would be substituted by that non-peptidic fragment adequately attached to the R₂, R₃, R₄ substituents as appearing in the formula I. (Angew. Chem. Int. Ed. 2015, 54, 11275-11278).

Another aspect of this invention relates to a pharmaceutical composition, which comprises a compound of formula **I** or a pharmaceutically acceptable salt thereof and one or more pharmaceutically acceptable excipients.

Another aspect of the invention relates to the compound of formula **I** or a pharmaceutical salt thereof for use as a medicament.

Another aspect of the invention relates to the compound of formula **I** or a pharmaceutical salt thereof for use in the treatment of a disease selected from cancer, an autoimmune disease, muscular dystrophy, emphysema, cachexia accompanying cancer or AIDS.

An another embodiment the invention relates to the compound of formula **I** or a pharmaceutical salt thereof for the use defined above, wherein cancer is a lymphoid malignancy, preferably selected from multiple myeloma (MM) including relapsed and refractory MM; non-Hodgkin lymphoma such as B-cell lymphomas including mantle cell lymphoma (MCL) and diffuse large B-celllymphoma (DLBCL), and Waldenstrom macroglobulinaemia.

In another embodiment the invention relates to the compound of formula **I** or a pharmaceutical salt thereof for the use defined above, wherein autoimmune disease is rheumatoid arthritis, systemic lupus erythematosus, Sjorgen's syndrome or scleroderma.

Another aspect of the present invention relates to the use of a compound of formula **I** or a pharmaceutically acceptable salt thereof for the manufacture of a medicament.

Another aspect of the present invention relates to the use of a compound of formula **I** or a pharmaceutically acceptable salt thereof for the manufacture of a medicament for the treatment of a disease selected from cancer, an autoimmune disease, muscular dystrophy, emphysema, cachexia accompanying cancer or AIDS.

Another aspect of the present invention relates to a method of treating a disease in a subject in need thereof, especially a human being, which comprises administering to said subject an effective amount of a compound of formula **I** or a pharmaceutically acceptable salt thereof.

Another aspect of the present invention relates to a method of treating a disease selected from cancer, an autoimmune disease, muscular dystrophy, emphysema, cachexia accompanying cancer or AIDS, in a subject in need thereof, especially a human being, which comprises administering to said subject an effective amount of a compound of formula **I** or a pharmaceutically acceptable salt thereof.

Another aspect of the present invention relates to a process for the preparation of a compound of formula **I** as defined above, which comprises:
The present invention also relates to a pharmaceutical composition that comprises a compound of the present invention (or a pharmaceutically acceptable salt or solvate thereof) and one or more pharmaceutically acceptable excipients. The excipients must be "acceptable" in the sense of being compatible with the other ingredients of the composition and not deleterious to the recipients thereof.

The compounds of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

Solid compositions for oral administration include tablets, granulates and capsules. In any case the manufacturing method is based on a simple mixture, dry granulation or wet granulation of the active compound with excipients. These excipients can be, for example, diluents such as lactose, microcrystalline cellulose, mannitol or calcium hydrogenphosphate; binding agents such as for example starch, gelatin or povidone; disintegrants such as sodium carboxymethyl starch or sodium croscarmellose; and lubricating agents such as for example magnesium stearate, stearic acid or talc. Tablets can be additionally coated with suitable excipients by using known techniques with the purpose of delaying their disintegration and absorption in the gastrointestinal tract and thereby provide a sustained action over a longer period, or simply to improve their organoleptic properties or their stability. The active compound can also be incorporated by coating onto inert pellets using natural or synthetic film-coating agents. Soft gelatin capsules are also possible, in which the active compound is mixed with water or an oily medium, for example coconut oil, mineral oil or olive oil.

Powders and granulates for the preparation of oral suspensions by the addition of water can be obtained by mixing the active compound with dispersing or wetting agents; suspending agents and preservatives. Other excipients can also be added, for example sweetening, flavouring and colouring agents.

Liquid forms for oral administration include emulsions, solutions, suspensions, syrups and elixirs containing commonly-used inert diluents, such as purified water, ethanol, sorbitol, glycerol, polyethylene glycols (macrogols) and propylene glycol. Said compositions can also contain coadjuvants such as wetting, suspending, sweetening, flavouring agents, preservatives and buffers.

Injectable preparations, according to the present invention, for parenteral administration, comprise sterile solutions, suspensions or emulsions, in an aqueous or non-aqueous solvent such as propylene glycol, polyethylene glycol or vegetable oils. These compositions can also contain coadjuvants, such as wetting, emulsifying, dispersing agents and preservatives. They may be sterilized by any known method or prepared as sterile solid compositions, which will be dissolved in water or any other sterile injectable medium immediately before use. It is also possible to start from sterile materials and keep them under these conditions throughout all the manufacturing process.

For the rectal administration, the active compound can be preferably formulated as a suppository on an oily base, such as for example vegetable oils or solid semisynthetic glycerides, or on a hydrophilic base such as polyethylene glycols (macrogol).

The compounds of the invention can also be formulated for their topical application for the treatment of pathologies occurring in zones or organs accessible through this route, such as eyes, skin and the intestinal tract. Formulations include creams, lotions, gels, powders, solutions and patches wherein the compound is dispersed or dissolved in suitable excipients.

For the nasal administration or for inhalation, the compound can be formulated as an aerosol, and it can be conveniently released using suitable propellants.

The dosage and frequency of doses will depend upon the nature and severity of the disease to be treated, the age, the general condition and body weight of the patient, as well as the particular compound administered and the route of administration, among other factors. A representative example of a suitable dosage range is from about 0.01 mg/Kg to about 100 mg/Kg per day, which can be administered as single or divided doses.

There is no limitation on the type of salt that can be used, provided that these are pharmaceutically acceptable when used for therapeutic purposes. The term pharmaceutically acceptable salt refers to those salts which are, according to medical judgment, suitable for use in contact with the tissues of humans and other mammals without undue toxicity, irritation, allergic response and the like. Pharmaceutically acceptable salts are well known in the art.

The salts of a compound of formula **I** can be obtained during the final isolation and purification of the compounds of the invention or can be prepared by treating a compound of formula **I** with a sufficient amount of the desired acid or base to give the salt in a conventional manner. The salts of the compounds of formula **I** can be converted into other salts of the compounds of formula **I** by ion exchange using ionic exchange resins.

The compounds of formula **I** and their salts may differ in some physical properties, but they are equivalent for the purposes of the present invention. All salts of the compounds of formula **I** are included within the scope of the invention.

The compounds of the present invention may form complexes with solvents in which they are reacted or from which they are precipitated or crystallized. These complexes are known as solvates. As used herein, the term solvate refers to a complex of variable stoichiometry formed by a solute (a compound of formula **I** or a salt thereof) and a solvent. Examples of solvents include pharmaceutically acceptable solvents such as water, ethanol and the like. A complex with water is known as a hydrate. Solvates of compounds of the invention (or salts thereof), including hydrates, are included within the scope of the invention.

The compounds of formula **I** may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula I, including all polymorphic forms ("polymorphs") thereof, are included within the scope of the invention.

Some of the compounds of the present invention may exist as several diastereoisomers and/or several optical isomers. Diastereoisomers can be separated by conventional techniques such as chromatography or fractional crystallization. Optical isomers can be resolved by conventional techniques of optical resolution to give optically pure isomers. This resolution can be carried out on any chiral synthetic intermediate or on products of formula I. Optically pure isomers can also be individually obtained using enantiospecific synthesis. The present invention covers all individual isomers as well as mixtures thereof (for example racemic mixtures or mixtures of diastereomers), whether obtained by synthesis or by physically mixing them.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

**Fig. 1****.** Competitive activity-based protein profiling (ABPP) assay of epoxyketones A, B, C and D against the fluorescent probes of catalytic subunits of proteasome.
**Fig. 2****.** Competitive activity-based protein profiling (ABPP) assay of epoxyketones B, BBn and BPy against the fluorescent probes of catalytic subunits of proteasome
**Fig. 3****.** Fluorescence progress curves for inhibitors BBn, BPy, as compared to already known epoxyketones against c20s proteasome.
**Fig. 4****.** Fluorescence progress curves for inhibitors BBn, BPy, as compared to already known epoxyketones against i20s proteasome.

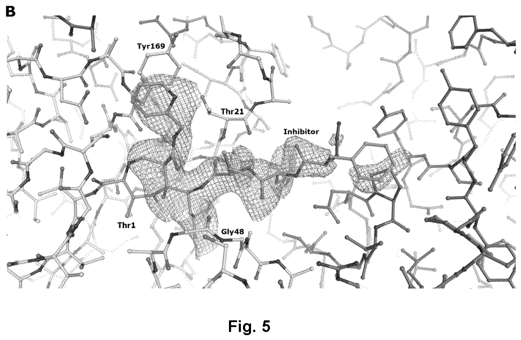

**Fig. 5****.** Snapshot of X-ray structure of the complex of inhibitor Bpy with c20S proteasome

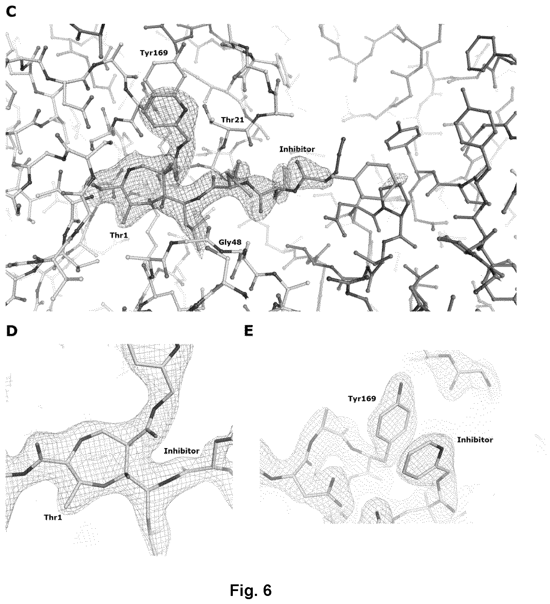

**Fig. 6****.** Snapshot of X-ray structure of the complex between the inhibitor Bpy and c20S proteasome

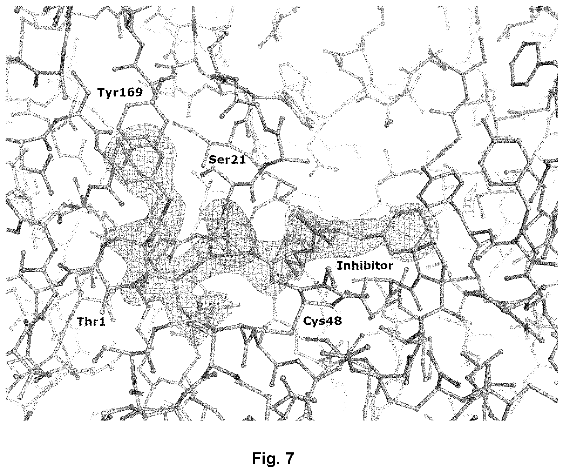

**Fig. 7****.** Snapshot of X-ray structure of the complex between the inhibitor Bpy and i20S proteasome

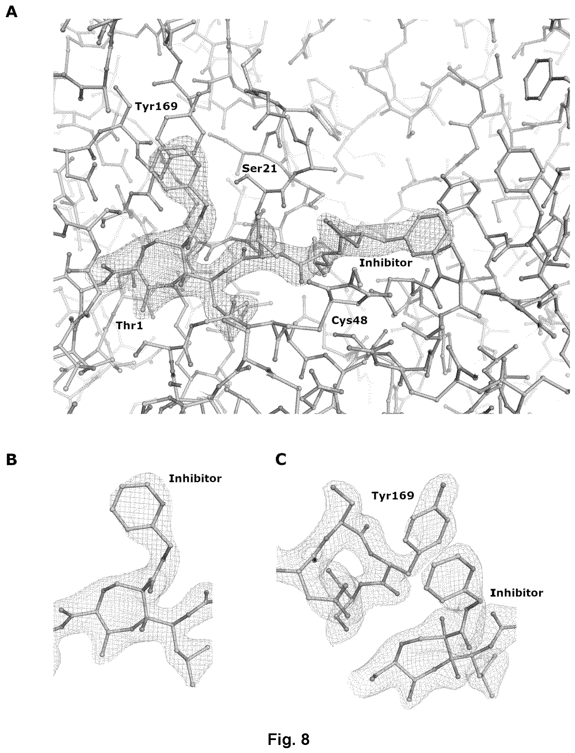

**Fig. 8****.** Snapshot of X-ray structure of the complex between inhibitor Bpy and i20S proteasome

### Examples

### Abbreviations

- Boc: Tert-butoxycarbonyl
- Boc₂O: Di-tert-butyl decarbonate
- t-Bu: Tert-butyl
- calcd: Calculated
- Cbz: Benzyloxycarbonyl
- CDCl₃: Chloroform deuterated
- COSY: Correlated Spectroscopy
- δ: Chemical shift
- d: Doublet
- DABCO: 1,4-Diazabicyclo[2.2.2] octane
- DCM: Dichloromethane
- dd: Double doublet
- ddt: Double double triplet
- ddq: Double double quadruplet
- DIBAL-H: Diisobutylaluminum hydride
- DiPEA: *N,N*-Diisopropylethylamine
- DMF: *N,N*-Dimethylformamide
- DMP: Dess-Martin Periodinane
- DMSO: Dimethyl sulfoxide
- DMSO-*d*₆: Hexadeuterodimethyl sulfoxide
- d.r.: Diastereomeric ratio
- dt: Double triplet
- dq: Double quadruplet
- EDC: *N*-(3-Dimethylaminopropyl)-*N*'-ethylcarbodiimide
- *ent*: Enantiomer
- equiv.: Equivalent
- ESI: Electrospray ionization
- Et₃N: Triethyl amine
- EtOH: Ethanol
- EtOAc: Ethyl acetate
- Fmoc: Fluorenemethoxycarbonyl
- g: Grams
- h: Hour
- Hex: Hexanes
- HOBt: 1-Hydroxybenzotriazole hydrate
- HRMS: High resolution mass spectra
- Hz: Hertz
- J: Coupling constant
- m: Multiplet
- Me: Methyl
- MeOH: Methanol
- MHz: Megahertz
- min: Minutes
- mg: Miligrams
- mL: Mililitres
- mmol: Milimoles
- MS: Mass spectrometry
- m/z: mass-to-charge ratio
- NMR: Nuclear magnetic resonance
- o.n.: Overnight
- ppm: Parts per milion
- Q: Quadrupole
- r.t.: Room temperature
- s: Singlet
- t: Triplet
- T: Temperature
- TBHP: tert-Butyl hydroperoxide
- TFA: Trifluoroacetic acid
- TLC: Thin Layer Chromatography
- TOF: Time of flight

### General Information

Unless otherwise specified, all reactions were carried out under nitrogen atmosphere with magnetic stirring. All solvents and reagents were obtained from commercial sources and were purified according to standard procedures before use. ¹H and ¹³C NMR spectra were measured in CDCl₃ (¹H, 7.27 ppm; ¹³C, 77.0 ppm) or DMSO-*d₆* (¹H, 2.50 ppm; ¹³C, 39.5 ppm) solution at 30 °C on a 300 or 400 MHz NMR spectrometer. Mass spectra were measured in a QTOF I (quadrupole-hexapole TOF) mass spectrometer with an orthogonal Z-spray-electrospray interface. EM Science Silica Gel 60 was used for column chromatography, while TLC was performed with precoated plates (Kieselgel 60, F₂₅₄, 0.25 mm).

*tert-Butyl* nitrite was synthesized using t-butanol and sodium nitrite following a procedure described by Coe.¹ And 4 M HCl solution in dioxane was prepared by bubbling HCl gas, produced from H₂SO₄ and NaCl, into freshly-distilled dioxane and it was titrated with NaOH 1 M through standard procedure.

### General Experimental Procedures

### Experimental procedure for the synthesis of the linker

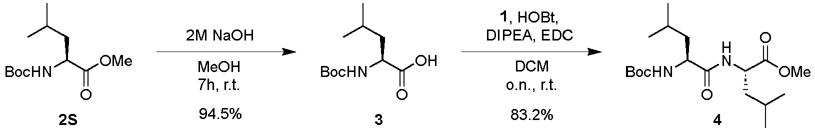

### (Tert-butoxycarbonyl)-L-leucine (3).

To an ice-bath cold solution of methyl (tert-butoxycarbonyl)-L-leucinate (2S) (5.517 g, 20 mmol, 1 equiv.) in MeOH (100 mL, 5 mL/mmol), an aqueous 2 M NaOH solution (20 mL, 40 mmol, 2 equiv.) was added dropwise. The resulting mixture was stirred for 7 h at room temperature and was monitored by TLC. Then, H₂O (100 mL, 5 mL/mmol) was added and the pH value was adjusted to pH = 2 using concentrated HCl. The protonated acid was extracted with DCM (5 x 25 mL, 1.25 mL/mmol), dried over MgSO₄ and concentrated under vacuum to afford a colourless oil that crystalized. The crude material was used without any further purification.

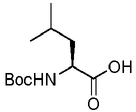

Colourless crystals (yield 4.367 g, 18.89 mmol, 94.5 %):
¹H NMR (400 MHz, CDCl₃) δ 4.86 (d, *J* = 5.3 Hz, 1H), 4.37 - 4.25 (m, 1H), 1.82 - 1.63 (m, 2H), 1.54 (ddd, *J* = 13.5, 9.3, 5.5 Hz, 1H), 1.45 (s, 9H), 0.99 - 0.94 (m, 6H) ppm;
¹³C NMR (101 MHz, CDCl₃) δ 177.47, 155.95, 80.49, 52.18, 41.48, 28.46, 24.96, 22.98, 21.96 ppm;

### Methyl (tert-butoxycarbonyl)-L-leucyl-L-leucinate (4).

To an ice-bath cold solution of methyl L-leucinate (1) (1.835 g, 10 mmol, 1 equiv.) in DCM (100 mL, 10 mL/mmol), 3 (2.570 g, 11 mmol, 1.1 equiv.) was added and stirred for 5 min. Then, HOBt (1.532 g, 11 mmol, 1.1 equiv.) was added and stirred for 5 min. Then, DIPEA (6.97 mL, 40 mmol, 4 equiv.) was added and stirred for 10 min. Finally, EDC (2.347 g, 12 mmol, 1.2 equiv.) was added and stirred overnight at room temperature and monitored by TLC. Then, the reaction mixture was quenched with a saturated aqueous solution of NH₄Cl (100 mL, 10 mL/mmol), extracted with DCM (3 x 25 mL, 3 x 2.5 mL/mmol), washed with 1 M HCl (25 mL, 2.5 mL/mmol), saturated aqueous solution of NaHCO₃ (25 mL, 2.5 mL/mmol), and brine (25 mL, 2.5 mL/mmol). Organic layers were dried over MgSO₄ and concentrated under vacuum. The crude material was purified by chromatography (silica gel, Hex/ACOEt 6:4 to 1:1) to afford the pure compound.

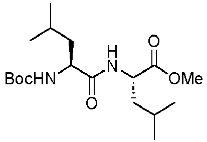

White solid (yield 2.983 g, 8.32 mmol, 83.2 %):
¹H NMR (400 MHz, CDCl₃) δ 6.39 (d, *J =* 7.8 Hz, 1H), 4.84 (s, 1H), 4.61 (td, J = 8.8, 4.8 Hz, 1H), 4.16 - 4.00 (m, 1H), 3.72 (s, 3H), 1.76 - 1.47 (m, 6H), 1.44 (s, 9H), 1.01 - 0.87 (m, 12H) ppm;
¹³C NMR (101 MHz, CDCl₃) δ 173.29, 172.33, 155.85, 80.25, 52.38, 50.78, 41.76, 28.42, 24.88, 24.84, 22.98, 21.98 ppm;
HRMS (ESI) *m*/*z* calcd for C₁₈H₃₄N₂O₅ [M+H]⁺: 359.2546, found: 359.2546.

### Experimental procedure for the synthesis of the "clickable" part

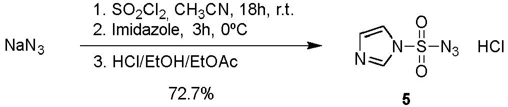

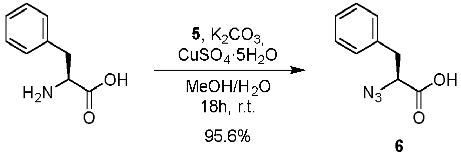

### 1H-imidazole-1-sulfonyl azide hydrochloride (5).

A suspension of NaN₃ (2,000 g, 30 mmol, 1 equiv.) in anhydrous CH₃CN (30 mL, 1 mL/mmol) was cooled with an ice-bath. Then, sulfuryl chloride (2.5 mL, 30 mmol, 1 equiv.) was added dropwise and the mixture was stirred overnight at room temperature. Imidazole (3.980 g, 60 mmol, 2 equiv.) was added slowly to the reaction cooled with an ice-bath, and the mixture was stirred for 3 h at room temperature (it was observed a colour change from white to coral-red). After this time, EtOAc (60 mL, 2 mL/mmol) was added and the reaction mixture was washed with H₂O (2 x 40 mL, 2 x 1.33 mL/mmol) and saturated aqueous NaHCO₃ solution (2 x 40 mL, 2 x 1.33 mL/mmol). The organic layer was dried over MgSO₄ and filtered. In a separate flask, an ice-bath solution of anhydrous HCl in ethanol was prepared by the dropwise addition of acetyl chloride (3.28 mL, 46 mmol, 1.53 equiv.) to dry ethanol (7.5 mL, 1.53 mL/mmol). The EtOAc solution was cooled with an ice-bath, and while stirring, the HCl solution in EtOH was added dropwise to precipitate a white solid. The precipitate was filtered, washed with EtOAc (3 x 15 mL, 3 x 0.5 mL/mmol), and dried under pressure to give imidazole-1-sulfonyl azide hydrochloride as a white solid. The crude material was used without any further purification.

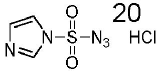

White solid (yield 4.574 g, 21.82 mmol, 72.7 %):
¹H NMR (400 MHz, D₂O) δ 9.39 (s, 1H), 8.08 (s, 1H), 7.67 (s, 1H) ppm;
¹³C NMR (101 MHz, DMSO-d6₃) δ 137.62, 131.25, 118.74 ppm;
HRMS (ESI) *m*/*z* calcd for C₃H₃N₅O₂S [M+H]⁺: 174.0086, found: 174.0089.

### (S)-2-azido-3-phenylpropanoic acid (6).

To a solution of *L*-phenylalanine (800.9 mg, 4.8 mmol, 1 equiv.) in MeOH:H₂O mixture (24/4.8 mL, 5/1 mL/mmol), K₂CO₃ (1.809 g, 13.0 mmol, 2.7 equiv.) and CuSO₄·5H₂O (12.1 mg, 0.048 mmol, 0.01 equiv.) were added. Then, ImSO₂N₃·HCl (5) (1.201 mg, 5.8 mmol, 1.2 equiv) was added. The resulting mixture was stirred for 18 h at room temperature while monitored by TLC. The reaction was concentrated under reduced pressure, diluted with H₂O (12 mL, 2.5 mL/mmol) and washed with DCM (3 x 8 mL, 3 x 1.67 mL/mmol). Then, the aqueous layer was acidified with 1 M HCl to pH = 1. It was observed a colour change from dark blue (pH = 8), blue-green (pH = 4), yellow-green to yellow (pH = 1). It was extracted with DCM (4 × 8 mL, 3 x 1.67 mL/mmol), washed with brine (8 mL) and the organic layer was dried over MgSO₄, filtered, and concentrated under vacuum. The crude material was used without any further purification.

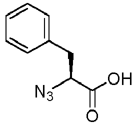

Yellowish oil (yield 877 mg, 4.59 mmol, 95.6 %):
¹H NMR (300 MHz, CDCl₃) δ 7.97 - 7.50 (m, 1H), 7.40 - 7.25 (m, 5H), 4.16 (dd, *J* = 8.9, 5.0 Hz, 1H), 3.25 (dd, *J* = 14.1, 5.0 Hz, 1H), 3.05 (dd, *J* = 14.1, 8.9 Hz, 1H) ppm;
¹³C NMR (101 MHz, CDCl₃) δ 175.11, 135.74, 129.36, 128.93, 127.59, 63.20, 37.67 ppm;
HRMS (ESI) *m*/*z* calcd for C₉H₉N₃O₂ [M-H]⁻: 190.0616, found: 190.0618.

### Experimental procedure for the coupling of the azido-L-phenylalanine to the linker and activation to acyl hydrazine

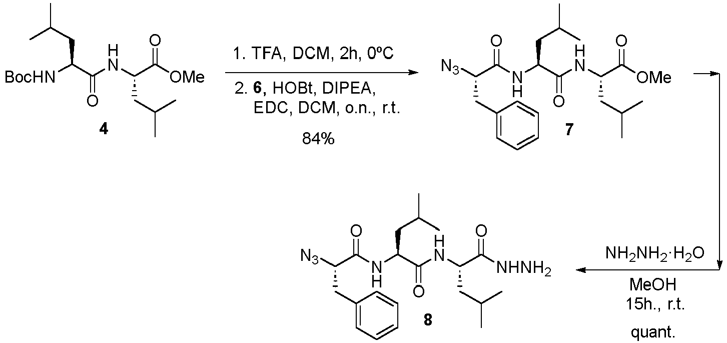

### Methyl ((S)-2-azido-3-phenylpropanoyl)-L-leucyl-L-leucinate (7).

To an ice-bath cold solution of 4 (1.463 g, 4.0 mmol, 1 equiv.) in DCM (11.36 mL, 2.84 mL/mmol), a solution of 1:1 TFA/DCM (6/6 mL, 1.5/1.5 mL/mmol) was added and stirred cold with an ice-bath until TLC showed starting material consumption (2 h).

Then, the reaction mixture was concentrated under vacuum. The crude material was used in the next step without any further purification.

The product was dissolved in DCM (40 mL, 10 mL/mmol) and stirred acold with an ice-bath. Then, 6 (858.4 mg, 4.4 mmol, 1.1 equiv.) was added and stirred for 5 min. After this time, HOBt (606.7 mg, 4.4 mmol, 1.1 equiv.) was added and stirred for 5 min. Then, DIPEA (2.71 mL, 16 mmol, 4 equiv.) was added and stirred for 10 min. Finally, EDC (936.4 mg, 4.8 mmol, 1.2 equiv.) was added and stirred overnight at room temperature. Then, the reaction mixture was quenched with a saturated aqueous solution of NH₄Cl (40 mL, 10 mL/mmol) extracted with DCM (3 x 27.2 mL, 3 x 6.8 mL/mmol), washed with 1 M HCl (6.8 mL, 2.5 mL/mmol), saturated aqueous solution of NaHCOs (27.2 mL, 2.5 mL/mmol), and brine (27.2 mL, 2.5 mL/mmol). Organic layers were dried over MgSO₄ and concentrated under vacuum. The crude material was purified by chromatography (silica gel, Hex/ACOEt 6:4 to 1:1) to afford the pure compound.

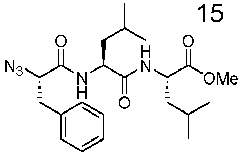

White solid (yield 1.450 g, 3.36 mmol, 84 %):
¹H NMR (400 MHz, CDCl₃) δ 7.27 - 7.18 (m, 5H), 7.14 (d, *J* = 7.9 Hz, 1H), 7.06 (d, *J=* 8.7 Hz, 1H), 4.63 - 4.55 (m, 1H), 4.55 - 4.48 (m, 1H), 4.16 (dd, *J* = 8.2, 4.1 Hz, 1H), 3.67 (s, 3H), 3.12 (ddd, *J* = 22.3, 14.1, 6.1 Hz, 2H), 1.69 - 1.35 (m, 6H), 0.95 - 0.76 (m, 12H) ppm;
¹³C NMR (75 MHz, CDCl₃) δ 173.15, 171.46, 168.62, 135.96, 129.55, 128.67, 127.28, 65.23, 52.31, 51.55, 50.91, 41.28, 41.11, 38.30, 24.89, 24.49, 22.88, 22.76, 22.17, 21.99 ppm;
HRMS (ESI) *m*/*z* calcd for C₂₂H₃₃N₅O₄ [M+H]⁺: 432.2611, found: 432.2620.

### (S)-2-((S)-2-azido-3-phenylpropanamido)-N-((S)-1-hydrazineyl-4-methyl-1-oxopentan-2-yl)-4-methylpentanamide (8).

To a solution of 7 (2.905 g, 6.73 mmol, 1 equiv.) in MeOH (57.9 mL, 8.6 mL/mmol), hydrazine monohydrate (9.8 mL, 201.9 mmol, 30 equiv.) was added and the mixture was stirred for 15 h at room temperature. Then, the solvent was evaporated under vacuum, and coevaporated with DCM (57.9 mL, 8.6 mL/mmol) to afford a white solid that was used without any further purification.

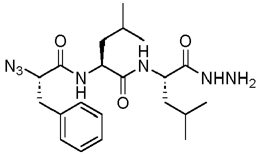

White solid: (yield 2.904 g, 6.73 mmol, quant.):
¹H NMR (300 MHz, CDCl₃) δ 7.50 (s, 1H), 7.35-7.22 (m, 5H), 6.62 (d, J= 7.3 Hz, 1H), 6.42 (d, *J=* 7.9 Hz, 1H), 4.45 - 4.28 (m, 2H), 4.27 (dd, *J* = 7.5, 4.2 Hz, 1H), 3.31 (dd, J = 14.1, 4.2 Hz, 1H), 3.08 (dd, *J* = 14.1, 7.5 Hz, 1H), 1.75 - 1.23 (m, 8H), 0.94 (d, *J* = 6.3 Hz, 3H), 0.90 (d, *J* = 6.3 Hz, 3H), 0.86 (d, *J* = 6.5 Hz, 3H), 0.83 (d, *J* = 6.5 Hz, 3H) ppm;
¹³C NMR (101 MHz, CDCl₃) δ 172.47, 171.79, 169.10, 135.92, 129.63, 128.83, 127.47, 65.31, 51.87, 50.55, 41.00, 40.85, 38.37, 24.97, 24.69, 23.03, 22.85, 22.27, 22.11 ppm;
HRMS (ESI) *m*/*z* calcd for C₂₁H₃₃N₇O₃ [M+Na]⁺: 454.2543, found: 454.2542.

### Experimental procedure for the synthesis of the warhead

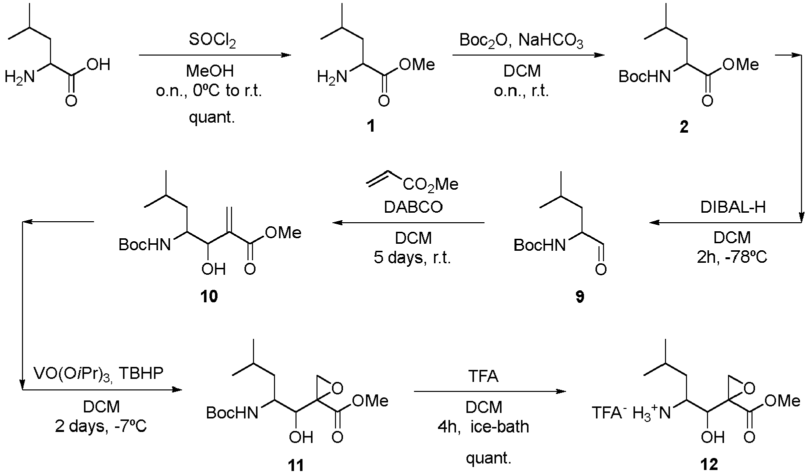

### Methyl-D-leucinate (1R).

To an ice-bath solution of D-Leucine (2.000 g, 15.1 mmol, 1 equiv.) in MeOH (37.8 mL, 2.5 mL/mmol), SOCl₂ (5 equiv.) was added dropwise, and the reaction was stirred from cold to room temperature. After 24 h or starting material consumption, the crude reaction was concentrated under vacuum. The product was used without further purification.

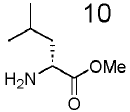

**1R.** White needles: (yield 2.192 g, 15.1 mmol, quant.):
¹H NMR (400 MHz, MeOD) δ 4.03 (dd, J = 8.6, 5.1 Hz, 1H), 3.84 (s, 3H), 1.86 - 1.74 (m, *J* = 13.3, 6.7 Hz, 1H), 1.86 - 1.60 (m, *J* = 27.2, 13.8, 6.9 Hz, 2H), 1.01 (d, *J* = 6.3 Hz, 3H), 0.99 (d, *J* = 6.2 Hz, 3H) ppm;
¹³C NMR (101 MHz, MeOD) δ 171.36, 53.73, 52.51, 40.67, 25.56, 22.55, 22.32 ppm; HRMS (ESI) m/z calcd for C₁₂H₁₅NO₂ [M+H]⁺: 146.1181, found: 146.1182.

### Methyl (tert-butoxycarbonyl)-L-leucinate (2S) and methyl (tert-butoxycarbonyl)-D-leucinate (2R).

To an ice-bath solution of methyl leucinate **(1S** or **1R)** (1 equiv.) in DCM (8.3 mL/mmol), Boc₂O (1.1 equiv.) and NaHCOs (2.2 equiv.) were added. The resulting mixture was stirred at room temperature overnight and the reaction was monitored by TLC. After completion, it was quenched by adding water (4.15 mL/mmol) and was extracted with DCM (3 x 1.25 mL/mmol), dried over Na₂SO₄ and concentrated under vacuum. The crude material was purified by chromatography (silica gel, Hex/ACOEt 7:3 to 6:4) to afford the pure compound or it was used without any further purification.

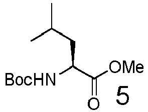

**2S.** Colourless oil (yield 11.826 g, 48.2 mmol, 95.4 %):
¹H NMR (400 MHz, CDCl₃) δ 4.92 (d, *J=* 6.1 Hz, 1H), 4.34 - 4.20 (m, 1H), 3.68 (s, 3H), 1.65 (tt, J= 12.9, 6.5 Hz, 1H), 1.60-1.51 (m, 1H), 1.50 - 1.42 (m, 1H), 1.39 (s, 9H), 0.90 (d, *J* = 6.5 Hz, 3H), 0.89 (d, *J* = 6.6 Hz, 3H) ppm;
¹³C NMR (101 MHz, CDCl₃) δ 174.03, 155.49, 79.80, 52.14, 41.90, 28.37, 24.83, 22.87, 21.95 ppm;
HRMS (ESI) *m*/*z* calcd for C₁₂H₂₃NO₄ [M+Na]⁺: 268.1525, found: 268.1528.

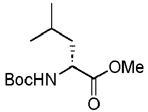

**2R.** Colourless oil (yield 3.364 g, 13.7 mmol, 90.1 % (2 steps)):
¹H NMR (400 MHz, CDCl₃) δ 4.90 (d, *J=* 6.0 Hz, 1H), 4.39 - 4.18 (m, *J =* 5.0 Hz, 1H), 3.70 (s, 3H), 1.73 - 1.52 (m, *J =* 21.4, 12.9, 7.1 Hz, 2H), 1.50 - 1.44 (m, 1H), 1.42 (s, 9H), 0.92 (d, *J* = 6.5 Hz, 3H), 0.91 (d, *J* = 6.6 Hz, 3H) ppm;
¹³C NMR (101 MHz, CDCl₃) δ 174.05, 155.50, 79.82, 52.15, 41.91, 28.37, 24.84, 22.87, 21.95 ppm;
HRMS (ESI) *m*/*z* calcd for C₁₂H₂₃NO₄ [M+Na]⁺: 268.1525, found: 268.1521.

### tert-Butyl (S)-(4-methyl-1-oxopentan-2-yl)carbamate (9S) and tert-butyl (R)-(4-methyl-1-oxopentan-2-yl)carbamate (9R).

To a -78 °C cold solution of compound **2S** or **2R** (1 equiv.) in DCM (3.5 mL/mmol), DIBAL-H (1.5 equiv.) was added and the mixture was stirred for 1 h. The reaction was monitored by TLC, adding more DIBAL-H (0.5 equiv.) if there was still starting material. The reaction was diluted by adding ethyl ether (2 mL/mmol), quenched with Rochelle salt solution (3.5 mL/mmol) and stirred for 1-2 h at room temperature until layers separation. The organic layer was extracted with Et₂O (3 x 1.25 mL/mmol), washed with brine (1.25 mL/mmol), dried over Na₂SO₄ and concentrated under vacuum. The crude material was purified by chromatography (silica gel, Hex/ACOEt 9:1 to 7:3) to afford the pure compound.

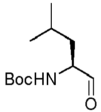

**9S.** Colourless oil (yield 2.429 g, 11.28 mmol, 63.5 %):
¹H NMR (400 MHz, CDCl₃) δ 9.58 (s, 1H), 4.92 (s, 1H), 4.23 (s, 1H), 1.85 - 1.71 (m, 1H), 1.71 -1.58 (m, 1H), 1.45 (s, 9H), 1.42 - 1.34 (m, 1H), 0.97 (d, *J* = 1.8 Hz, 3H), 0.95 (d, *J* = 2.0 Hz, 3H) ppm;
¹³C NMR (101 MHz, CDCl₃) δ 200.48, 155.76, 80.20, 58.58, 38.32, 28.42, 24.82, 23.21, 22.09 ppm;
HRMS (ESI) *m*/*z* calcd for C₁₁H₂₁NO₃ [M+Na]⁺: 238.1419, found: 238.1461.

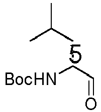

**9R.** Colourless oil (yield 1.662 g, 7.72 mmol, 64.3 %):
¹H NMR (300 MHz, CDCl₃) δ 9.53 (s, 1H), 5.04 (s, 1H), 4.18 (d, *J=* 4.1 Hz, 1H), 1.85 - 1.65 (m, 1H), 1.65 - 1.54 (m, 1H), 1.40 (s, 9H), 1.33 - 1.26 (m, 1H), 1.00 - 0.86 (m, 6H) ppm;
¹³C NMR (101 MHz, CDCl₃) δ 200.57, 155.76, 80.14, 58.52, 38.19, 28.38, 28.33, 24.76, 23.17, 22.02 ppm;
HRMS (ESI) *m*/*z* calcd for C₁₁H₂₁NO₃ [M+H]⁺: 216.1604, found: 216.1600.

To a solution of **9S** or **9R** (4.820 g, 22.388 mmol, 1 equiv.) in DCM (8.96 mL, 0.4 mL/mmol), methyl acrylate (8.146 mL, 89.552 mmol, 4 equiv.) and DABCO (2.563 g, 22.388 mmol, 1 equiv.) were added. The mixture was stirred (under N₂ atmosphere) for 5-7 days at room temperature. After the reaction completion (monitored by TLC), the mixture was diluted with DCM (78.4 mL, 3.5 mL/mmol) and quenched by adding 1 M HCl (40.3 mL, 1.8 mL/mmol). Then, it was extracted with DCM (2 x 78.4 mL, 2 x 3.5 mL/mmol) and organic layers were washed with brine (145.5 mL, 6.5 mL/mmol), dried over MgSO₄, and concentrated under vacuum. The crude material was purified by chromatography (silica gel, Hex/ACOEt 8:2 to 5:5) to afford the pure compound as 7:3 diastereomeric mixture (syn + anti).

### Methyl (3S,4S)-4-((tert-butoxycarbonyl)amino)-3-hydroxy-6-methyl-2-methyleneheptanoate (ent 10 (syn))

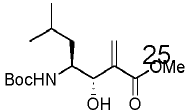

***ent*** 10 (syn + anti). Colourless oil:
¹H NMR (400 MHz, CDCl₃) (83:17 diastereomeric mixture) δ 6.30 (s, 1H), 5.90 (s, 1H), 4.67 (d, *J =* 8.0 Hz, 1H), 4.50 (s, 1H), 3.90 - 3.81 (m, 1H), 3.77 (s, 3H), 1.75 -1.60 (m, 1H), 1.53 (ddd, *J* = 15.2, 9.3, 5.9 Hz, 1H), 1.47 - 1.41 (m, 1H), 1.38 (s, 9H), 0.94 (d, *J* = 6.7 Hz, 3H), 0.92 (d, *J* = 6.6 Hz, 3H) ppm;
¹³C NMR (75 MHz, CDCl₃) (83:17 diastereomeric mixture) δ 166.77, 156.54, 140.87, 125.83, 79.37, 72.70, 53.21, 52.00, 41.35, 28.41, 25.13, 23.04, 22.43 ppm;
HRMS (ESI) *m*/*z* calcd for C₁₅H₂₇NO₅ [M+Na]⁺: 324.1787, found: 324.1789.

### Methyl (3R,4R)-4-((tert-butoxycarbonyl)amino)-3-hydroxy-6-methyl-2-methyleneheptanoate (10 (syn)).

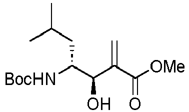

**10 (syn + anti).** Colourless oil:
¹H NMR (300 MHz, CDCl₃) (79:21 diastereomeric mixture) δ 6.29 (s, 1H), 5.89 (s, 1H), 4.72 (d, *J =* 9.8 Hz, 1H), 4.48 (s, 1H), 3.91 - 3.78 (m, 1H), 3.75 (s, *J =* 2.3 Hz, 3H), 2.89 (s, 1H), 1.66 (ddq, J = 18.9, 12.5, 6.4 Hz, 1H), 1.58 - 1.41 (m, 2H), 1.36 (s, 9H), 0.93 (d, *J* = 6.7 Hz, 3H), 0.91 (d, *J* = 6.6 Hz, 3H) ppm;
¹³C NMR (75 MHz, CDCl₃) (79:21 diastereomeric mixture) δ 166.74, 156.50, 140.89, 125.77, 79.30, 72.47, 53.10, 51.95, 41.36, 28.42, 25.09, 23.00, 22.41 ppm;
HRMS (ESI) *m*/*z* calcd for C₁₅H₂₇NO₅ [M+Na]⁺: 324.1787, found: 324.1789.

### Methyl (3S,4R)-4-((tert-butoxycarbonyl)amino)-3-hydroxy-6-methyl-2-methyleneheptanoate (10R (anti)).

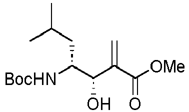

Colourless oil:
¹H NMR (400 MHz, CDCl₃) (rotamers mixture) δ 6.30 (s, 1H), 5.86 (t, *J =* 1.2 Hz, 1H), 4.56 (d, *J =* 8.1 Hz, 1H), 4.41 (s, 1H), 3.83 (dddd, J = 11.0, 8.9, 5.2, 3.3 Hz, 1H), 3.77 (s, 3H), 3.72 (s, 1H), 1.72 - 1.58 (m, 1H), 1.41 (s, *J =* 13.6 Hz, 9H), 1.37 - 1.32 (m, *J =* 3.0 Hz, 1H), 1.29 - 1.16 (m, 1H), 0.90 (d, *J* = 6.7 Hz, 3H), 0.86 (d, *J* = 6.5 Hz, 3H) ppm;
¹³C NMR (101 MHz, CDCl₃) δ 167.23, 156.39, 139.80, 127.25, 79.63, 75.35, 53.18, 52.06, 39.07, 28.45, 24.95, 23.77, 21.67 ppm;
HRMS (ESI) *m*/*z* calcd for C₁₅H₂₇NO₅ [M+Na]⁺: 324.1787, found: 324.1789.

### Methyl (3R,4S)-4-((tert-butoxycarbonyl)amino)-3-hydroxy-6-methyl-2-methyleneheptanoate (10S (anti)).

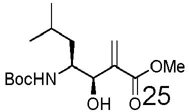

Colourless oil:
¹H NMR (400 MHz, CDCl₃) δ 6.26 (s, 1H), 5.87 (s, 1H), 4.76 (d, *J* = 9.3 Hz, 1H), 4.47 (s, 1H), 3.86 - 3.78 (m, 1H), 3.73 (s, 3H), 3.59 (s, 1H), 1.72 - 1.57 (m, 1H), 1.52 - 1.44 (m, 1H), 1.34 (s, 9H), 1.27 - 1.13 (m, 1H), 0.91 (d, *J* = 6.6 Hz, 3H), 0.89 (d, *J* = 6.5 Hz, 3H) ppm;
HRMS (ESI) *m*/*z* calcd for C₁₅H₂₇NO₅ [M+Na]⁺: 324.1787, found: 324.1789.

To a -7 °C cold solution of 10 (1 equiv.) in DCM (12.5 mL/mmol), VO(O*i*Pr)₃ (0.2 equiv.) and TBHP (5 equiv.) were added. The reaction mixture was stirred at -7 °C for 2 days and was monitored by TLC. Then, saturated aqueous Na₂S₂O₃ solution (12.5 mL/mmol) was added and the mixture was stirred for 15-30 min. Then, it was extracted with DCM (3 x 12.5 mL/mmol), washed with saturated aqueous Na₂S₂O₃ solution (12.5 mL/mmol), dried over MgSO₄, filtered and concentrated under vacuum. The crude material was purified by chromatography (silica gel, Hex/ACOEt 7:3 to 6:4) to afford the pure compound.

### Methyl (S)-2-((1R,2S)-2-((tert-butoxycarbonyl)amino)-1-hydroxy-4-methylpentyl)oxirane-2-carboxylate (ent 11 (syn-syn))

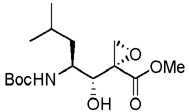

*ent* **11 (syn-syn + syn-anti).** Colourless oil (yield 378 mg (85:15 diastereomeric mixture), 1.25 mmol, 87.6 %):
¹H NMR (400 MHz, CDCl₃) (rotamers mixture) δ 4.58 (d, *J* = 9.8 Hz, 1H), 4.21 (dd, J= 15.6, 9.2 Hz, 1H), 4.13 (s, 1H), 3.78 (s, 3H), 3.17 (d, *J=* 6.0 Hz, 1H), 3.07 (d, *J* = 6.0 Hz, 1H), 2.32 (d, *J* = 1.3 Hz, 1H), 1.75 - 1.64 (m, 1H), 1.63 - 1.42 (m, 2H), 1.40 (s, 9H), 0.96 (d, *J* = 6.7 Hz, 3H), 0.93 (d, *J* = 6.6 Hz, 3H) ppm;
¹³C NMR (101 MHz, CDCl₃) δ 169.28, 156.01, 79.38, 69.29, 56.55, 52.83, 49.33, 48.25, 41.98, 28.47, 24.97, 22.91, 22.34 ppm;
HRMS (ESI) *m*/*z* calcd for C₁₅H₂₇NO₆ [M+Na]⁺: 340.1736, found: 340.1740.

### Methyl (R)-2-((1S,2R)-2-((tert-butoxycarbonyl)amino)-1-hydroxy-4-methylpentyl)oxirane-2-carboxylate (11 (syn-syn)).

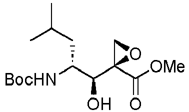

**11** (syn-syn + syn-anti). Colourless oil (yield 1.388 mg (85:15 diastereomeric mixture), 4.37 mmol, 66.1 %):
¹H NMR (300 MHz, CDCl₃) (rotamers mixture) δ 4.57 (d, *J* = 9.8 Hz, 1H), 4.21 (dd, J= 15.8, 9.0 Hz, 1H), 4.13 (s, 1H), 3.79 (s, 3H), 3.18 (d, *J=* 5.9 Hz, 1H), 3.08 (d, *J* = 6.0 Hz, 1H), 2.28 (s, *J* = 0.9 Hz, 1H), 1.76 - 1.65 (m, 1H), 1.63 - 1.45 (m, 2H), 1.40 (s, 9H), 0.96 (d, *J* = 6.6 Hz, 3H), 0.94 (d, *J* = 6.5 Hz, 3H) ppm;
¹³C NMR (75 MHz, CDCl₃) δ 169.28, 156.01, 79.39, 69.28, 56.54, 52.84, 49.33, 48.27, 41.98, 28.48, 24.97, 22.92, 22.35 ppm;
HRMS (ESI) *m*/*z* calcd for C₁₅H₂₇NO₆ [M+Na]⁺: 340.1736, found: 340.1740.

### Methyl (R)-2-((1R,2S)-2-((tert-butoxycarbonyl)amino)-1-hydroxy-4-methylpentyl)oxirane-2-carboxylate 11R ((syn-anti)).

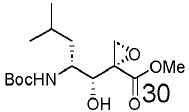

Hex/EtOAc 7:3 to 6:4. Colourless oil (yield 216 mg, 0.681 mmol):
¹H NMR (400 MHz, CDCl₃) δ 8.53 (ddd, J= 4.9, 1.6, 0.9 Hz, 1H), 7.65 (td, *J* = 7.7, 1.8 Hz, 1H), 7.61 (s, 1H), 7.24 - 7.15 (m, 2H), 4.70 - 4.62 (m, 1H), 4.59 (d, *J* = 5.7 Hz, 1H), 4.45 (d, *J* = 4.3 Hz, 1H), 4.41 (d, *J* = 4.6 Hz, 1H), 4.10 (tdd, J= 9.9, 4.9, 2.6 Hz, 1H), 3.53 (d, *J =* 4.2 Hz, 1H), 3.07 (d, *J =* 5.2 Hz, 1H), 2.96 (d, *J* = 5.2 Hz, 1H), 1.64 - 1.60 (m, 1H), 1.59 - 1.50 (m, 1H), 1.37 (s, 9H), 1.35 - 1.30 (m, 1H), 0.92 (d, *J* = 6.6 Hz, 3H), 0.91 (d, *J* = 6.5 Hz, 3H) ppm;
¹³C NMR (101 MHz, CDCl₃) δ 170.13, 155.77, 79.57, 74.23, 56.76, 52.83, 51.50, 49.65, 40.90, 28.47, 24.75, 23.86, 21.67 ppm;
HRMS (ESI) *m*/*z* calcd for C₁₅H₂₇NO₆ [M+Na]⁺: 340.1736, found: 340.1740.

### Methyl (S)-2-((1S,2R)-2-((tert-butoxycarbonyl)amino)-1-hydroxy-4-methylpentyl)oxirane-2-carboxylate (11S (syn-anti)).

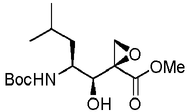

Hex/EtOAc 7:3 to 6:4. Colourless oil (yield 987 mg, 3.11 mmol):
¹H NMR (400 MHz, CDCl₃) δ 4.58 (d, *J* = 9.5 Hz, 1H), 4.02 - 3.87 (m, 1H), 3.74 (s, 3H), 3.66 - 3.59 (m, 1H), 3.06 (d, *J* = 5.7 Hz, 1H), 2.97 (d, *J* = 5.7 Hz, 1H), 1.64 (ddd, *J* = 9.6, 8.6, 5.0 Hz, 1H), 1.53 - 1.42 (m, 1H), 1.38 (s, 9H), 1.33 - 1.20 (m, 1H), 0.88 (d, *J* = 6.6 Hz, 3H), 0.87 (d, *J* = 6.3 Hz, 3H) ppm;
¹³C NMR (75 MHz, CDCl₃) δ 170.15, 155.78, 79.59, 74.30, 56.74, 52.85, 51.52, 49.68, 40.91, 28.48, 24.76, 23.87, 21.67 ppm.

To an ice-bath cold solution of 11 (1 equiv.) in DCM (2.84 mL/mmol), a solution of 1:1 TFA/DCM (1.5/1.5 mL/mmol) was added and stirred cold with an ice-bath until TLC showed starting material consumption (4h). Then, the reaction mixture was concentrated under vacuum. The crude material was used without any further purification.

### Experimental procedure for the coupling and oxidation of final compounds

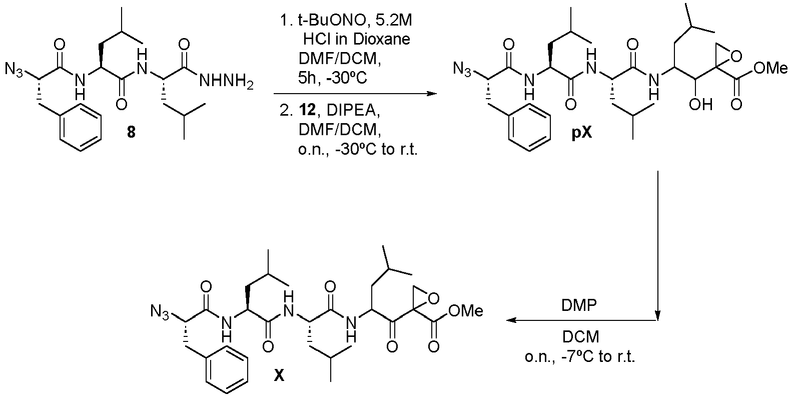

To a -30°C cold solution of 8 (1 equiv.) in 1:1 DMF/DCM solution (3.5 mL/mmol), *tert-*butyl nitrite (1.1 equiv.) and HCl 4 N in dioxane (2.8 equiv.) were added. The resulting mixture was stirred for 3 h at -30 °C or until TLC analysis showed complete consumption of the starting material.

Then, a mixture of deprotected epoxyalcohol 12 (syn-syn) and 12 (syn-anti) (1 equiv.) and DiPEA (1 equiv.) in DMF (2.5 mL/mmol), and DiPEA (3.8 equiv.) in DCM (2.5 mL/mmol) were added at -30 °C. Additional 1 mL DMF/ DCM mixture were used to dissolve and to add the epoxyalcohol to the reaction mixture. After the addition, the reaction mixture was allowed to warm up to room temperature overnight (16h). It was diluted with EtOAc (20 mL/mmol), quenched with H₂O (20 mL/mmol), and extracted with EtOAc (6 x 10 mL/mmol). Then, the organic layers were dried over Na₂SO₄ and concentrated under vacuum. The crude material was purified by flash chromatography (silica gel, Hex/ACOEt 6:4 to 1:1). Product was obtained as a diastereomeric mixture (yield 1.329 g, 2.155 mmol, 59.7 %), denoting racemization of the leucinal on the previous MBH step. Similar results were observed in both routes.

### Methyl (R)-2-((1S,2R)-2-((S)-2-((S)-2-((S)-2-azido-3-phenylpropanamido)-4-methylpentanamido)-4-methylpentanamido)-1-hydroxy-4-methylpentyl)oxirane-2-carboxylate (pA)

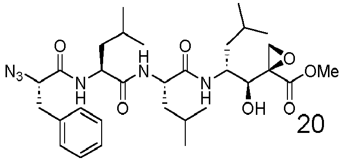

White solid
¹H NMR (400 MHz, CDCl₃) δ 7.31 - 7.20 (m, 5H), 6.96 - 6.83 (m, 2H), 6.61 (d, *J=* 9.4 Hz, 1H), 4.54 -4.40 (m, 3H), 4.29 (dd, *J* = 7.9, 4.1 Hz, 1H), 4.20 (d, *J=* 1.6 Hz, 1H), 3.73 (s, 3H), 3.28 (s, 1H), 3.25 (dd, *J =* 14.1, 4.1 Hz, 1H), 3.02 (dd, *J* = 14.3, 7.7 Hz, 1H), 3.02 - 2.98 (m, 2H), 1.59 (m, 4H), 1.53 -1.43 (m, 4H), 1.40 - 1.32 (m, 1H), 0.95 (d, *J* = 6.4 Hz, 3H), 0.93 - 0.86 (m, 9H), 0.85 (d, *J* = 6.6 Hz, 3H), 0.83 (d, *J* = 6.4 Hz, 3H) ppm;
¹³C NMR (101 MHz, CDCl₃) δ 171.98, 171.58, 169.42, 169.11, 135.90, 129.57, 128.73, 128.69, 127.30, 69.17, 65.06, 56.87, 52.71, 51.95, 51.87, 48.33, 48.21, 41.87, 41.53, 41.06, 38.22, 24.89, 24.56, 22.98, 22.95, 22.82, 22.34, 22.15, 21.98 ppm;
HRMS (ESI) *m*/*z* calcd for C₃₁H₄₈N₆O₇ [M+H]⁺: 617.3663, found: 617.3664.

### Methyl (S)-2-((1R,2S)-2-((S)-2-((S)-2-((S)-2-azido-3-phenylpropanamido)-4-methylpentanamido)-4-methylpentanamido)-1-hydroxy-4-methylpentyl)oxirane-2-carboxylate (pB).

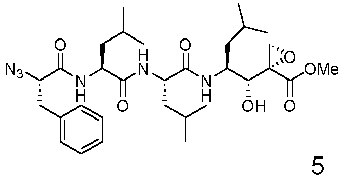

White solid
¹H NMR (400 MHz, CDCl₃) δ 7.35 - 7.21 (m, 5H), 6.59 (d, *J=* 7.7 Hz, 1H), 6.32 (d, *J=* 7.8 Hz, 1H), 6.20 (d, *J =* 9.3 Hz, 1H), 4.51 (dd, *J* = 14.8, 9.0 Hz, 1H), 4.36 - 4.27 (m, 2H), 4.25 (dd, *J* = 7.5, 4.1 Hz, 1H), 4.20 - 4.14 (m, 1H), 3.79 (s, 3H), 3.31 (dd, *J =* 14.1, 4.1 Hz, 1H), 3.16 (d, *J=* 5.8 Hz, 1H), 3.08 (d, *J=* 5.7 Hz, 1H), 3.07 (dd, J= 14.1, 7.6 Hz, 1H), 2.38 (dd, *J =* 3.0, 1.1 Hz, 1H), 1.75 - 1.67 (m, 1H), 1.64 - 1.45 (m, 6H), 1.45 - 1.32 (m, 2H), 0.96 (d, *J* = 6.2 Hz, 3H), 0.94 - 0.88 (m, 9H), 0.87 (d, *J* = 6.4 Hz, 3H), 0.84 (d, *J* = 6.4 Hz, 3H) ppm;
¹³C NMR (101 MHz, CDCl₃) δ 171.66, 171.58, 169.34, 169.11, 135.74, 129.68, 128.87, 127.53, 69.37, 65.43, 56.66, 52.88, 52.27, 52.01, 48.41, 48.28, 41.90, 40.64, 40.55, 38.43, 25.04, 24.98, 24.71, 23.11, 23.05, 22.93, 22.24, 22.17, 21.90 ppm; HRMS (ESI) *m*/*z* calcd for C₃₁H₄₈N₆O₇ [M+H]⁺: 617.3663, found: 617.3668.

### Methyl (R)-2-((1R,2S)-2-((S)-2-((S)-2-((S)-2-azido-3-phenylpropanamido)-4-methylpentanamido)-4-methylpentanamido)-1-hydroxy-4-methylpentyl)oxirane-2-carboxylate (pC).

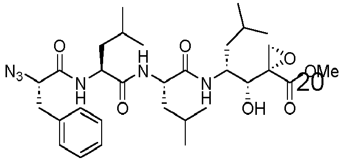

White solid:
¹H NMR (400 MHz, CDCl₃) δ 7.35 - 7.19 (m, 5H), 6.63 (d, *J=* 7.0 Hz, 1H), 6.42 (d, *J=* 8.5 Hz, 1H), 6.36 (d, *J=* 9.7 Hz, 1H), 4.43 (td, *J* = 9.0, 5.5 Hz, 1H), 4.39 - 4.31 (m, 1H), 4.31 - 4.24 (m, 1H), 4.28 (dd, *J* = 7.5, 4.2 Hz, 1H), 3.78 (s, 3H), 3.58 (t, *J* = 7.0 Hz, 1H), 3.29 (dd, *J* = 14.1, 4.1 Hz, 1H), 3.17 (d, *J* = 7.3 Hz, 1H), 3.06 (dd, *J* = 14.5, 6.7 Hz, 1H), 3.04 (d, *J* = 5.9 Hz, 1H), 2.97 (d, *J* = 5.8 Hz, 1H), 1.74 (ddd, *J* = 13.8, 8.4, 5.5 Hz, 1H), 1.66 - 1.52 (m, 3H), 1.51 - 1.31 (m, 5H), 0.92 (d, *J* = 6.7 Hz, 3H), 0.92 (d, *J* = 6.5 Hz, 3H), 0.90 (d, *J* = 3.1 Hz, 3H), 0.88 (d, *J* = 3.2 Hz, 3H), 0.87 (d, *J* = 6.8 Hz, 3H), 0.83 (d, *J* = 6.4 Hz, 3H) ppm;
¹³C NMR (101 MHz, CDCl₃) δ 171.58, 171.44, 170.09, 169.42, 135.82, 129.63, 128.81, 127.45, 73.67, 65.39, 56.88, 52.92, 52.55, 51.85, 50.04, 49.72, 40.76, 40.73, 39.87, 38.35, 25.04, 24.74, 24.73, 23.91, 23.08, 23.07, 21.99, 21.95, 21.51 ppm;
HRMS (ESI) *m*/*z* calcd for C₃₁H₄₈N₆O₇ [M+H]⁺: 617.3663, found: 617.3664.

### Methyl (S)-2-((1S,2R)-2-((S)-2-((S)-2-((S)-2-azido-3-pheny)propanamido)-4-methylpentanamido)-4-methylpentanamido)-1-hydroxy-4-methylpentyl)oxirane-2-carboxylate (pD).

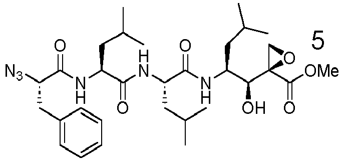

White solid:
¹H NMR (400 MHz, CDCl₃) δ 7.34 - 7.21 (m, 5H), 6.68 (d, *J =* 8.2 Hz, 1H), 6.49 (d, *J =* 8.1 Hz, 1H), 6.27 (d, *J =* 9.6 Hz, 1H), 4.43 - 4.28 (m, 3H), 4.23 (dd, J = 7.7, 4.2 Hz, 1H), 3.78 (s, 3H), 3.70 (t, *J* = 6.3 Hz, 1H), 3.30 (dd, *J =* 14.1, 4.1 Hz, 1H), 3.09 (d, *J =* 5.8 Hz, 1H), 3.06 (dd, J = 14.0, 7.6 Hz, 1H), 3.00 (d, *J =* 5.8 Hz, 1H), 2.91 (d, *J* = 6.4 Hz, 1H), 1.73 - 1.65 (m, 1H), 1.64 - 1.57 (m, 1H), 1.55 - 1.47 (m, 3H), 1.46 - 1.30 (m, 4H), 0.93 (d, *J* = 6.4 Hz, 3H), 0.90 (d, *J* = 6.1 Hz, 3H), 0.89 (d, *J* = 6.5 Hz, 3H), 0.87 (d, *J* = 6.9 Hz, 3H), 0.85 (d, *J* = 6.7 Hz, 3H), 0.84 (d, *J* = 6.4 Hz, 3H) ppm;
¹³C NMR (101 MHz, CDCl₃) δ 171.68, 171.35, 169.91, 168.79, 135.91, 129.66, 128.80, 127.43, 73.19, 65.37, 56.69, 52.90, 52.21, 51.74, 49.76, 49.59, 40.98, 40.66, 39.97, 38.38, 24.87, 24.66, 24.61, 23.96, 22.98, 22.27, 22.14, 21.52 ppm;
HRMS (ESI) *m*/*z* calcd for C₃₁H₄₈N₆O₇ [M+H]⁺: 617.3663, found: 617.3664.

To an ice-bath cold solution of pX (126 mg, 0.397 mmol, 1 equiv.) in DCM (3.97 mL, 10 mL/mmol), DMP (260.4 mg, 0.596 mmol, 1.5 equiv.) was added. The resulting mixture was stirred for 3 h cold with an ice-bath and monitored by TLC. As it was required, more DMP (0.2 equiv) was added and stirred for additional 40 min cold with an ice-bath until alcohol disappearance (TLC). After that, Et₂O (24 mL, 60 mL/mmol) and 100/120 g/L Na₂S₂O₃/NaHO₃ aqueous solution (24 mL, 60 mL/mmol) were added and stirred until phase separation was observed (15 min). The aqueous layer was extracted with ether (3 x 24 mL, 60 mL/mmol), washed with an aqueous solution of 100/120 g/L Na₂S₂O₃/NaHCO₃ (24 mL, 60 mL/mmol), dried over Na₂SO₄ and concentrated under vacuum. The crude material was used without any further purification.

### Methyl (R)-2-(((S)-2-azido-3-phenylpropanoyl)-L-leucyl-L-leucyl-D-leucyl)oxirane-2-carboxylate (A)

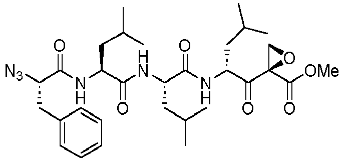

White solid (yield 165.8 mg, 0.269 mmol, 83.3 %):
¹H NMR (400 MHz, CDCl₃) δ 7.34 - 7.21 (m, 5H), 6.66 (d, *J=* 8.3 Hz, 1H), 6.57 (d, *J* = 6.8 Hz, 1H), 6.27 (d, *J =* 8.1 Hz, 1H), 5.02 (ddd, *J* = 10.4, 8.3, 3.3 Hz, 1H), 4.42 (ddd, J = 9.0, 8.3, 5.4 Hz, 1H), 4.32 (dd, *J* = 7.4, 4.2 Hz, 1H), 4.24 (ddd, J= 9.3, 6.9, 5.6 Hz, 1H), 3.84 (s, 3H), 3.31 (dd, *J=* 14.1, 4.1 Hz, 1H), 3.25 (d, *J* = 6.4 Hz, 1H), 3.13 (d, *J=* 6.4 Hz, 1H), 3.07 (dd, *J=* 14.1, 7.5 Hz, 1H), 1.76 (ddd, *J* = 13.7, 8.3, 5.4 Hz, 1H), 1.64 - 1.55 (m, 4H), 1.52 - 1.35 (m, 4H), 0.96 - 0.92 (m, 9H), 0.90 (d, *J* = 6.5 Hz, 3H), 0.88 (d, *J* = 6.5 Hz, 3H), 0.84 (d, *J* = 6.5 Hz, 3H) ppm;
¹³C NMR (101 MHz, CDCl₃) δ 200.52, 171.74, 169.39, 166.99, 135.86, 129.64, 128.82, 127.45, 65.27, 58.56, 54.88, 53.40, 52.26, 51.73, 39.28, 38.34, 25.02, 24.74, 23.44, 22.99, 22.04, 21.45 ppm;
HRMS (ESI) *m*/*z* calcd for C₃₁H₄₈N₆O₇ [M+Na]+: 637.3326, found: 637.3326.

### and methyl (S)-2-(((S)-2-azido-3-phenylpropanoyl)-L-leucyl-L-leucyl-L-leucyl)oxirane-2-carboxylate (B).

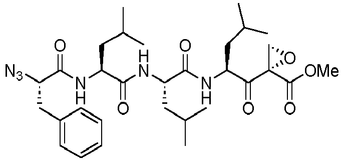

White solid (yield 38 mg, 0.062 mmol, 79.5 %):
¹H NMR (400 MHz, CDCl₃) δ 7.33 - 7.19 (m, 5H), 6.88 (d, *J=* 8.4 Hz, 1H), 6.83 (d, *J=* 8.2 Hz, 1H), 6.76 (d, *J =* 8.1 Hz, 1H), 5.01 (ddd, *J* = 10.5, 8.5, 3.5 Hz, 1H), 4.50 - 4.37 (m, 2H), 4.24 (dd, J = 7.8, 4.2 Hz, 1H), 3.82 (s, 3H), 3.31 - 3.24 (m, 1H), 3.24 (d, *J=* 6.3 Hz, 1H), 3.11 (d, *J=* 6.3 Hz, 1H), 3.05 (dd, *J=* 14.1, 7.8 Hz, 1H), 1.74 - 1.68 (m, 1H), 1.68 - 1.56 (m, 3H), 1.54 - 1.36 (m, 5H), 0.91 (d, *J =* 6.4 Hz, 3H), 0.90 (d, *J* = 6.4 Hz, 6H), 0.88 (d, *J* = 6.7 Hz, 3H), 0.85 (d, *J* = 6.7 Hz, 3H), 0.83 (d, *J* = 6.6 Hz, 3H) ppm;
¹³C NMR (101 MHz, CDCl₃) δ 200.43, 171.75, 171.51, 169.02, 166.96, 135.80, 129.69, 128.85, 127.51, 65.43, 58.61, 54.79, 53.45, 52.08, 52.03, 51.90, 40.64, 40.36, 39.63, 38.41, 24.93, 24.66, 23.49, 22.99, 22.93, 22.19, 22.11, 21.48 ppm;
HRMS (ESI) *m*/*z* calcd for C₃₁H₄₈N₆O₇ [M+Na]⁺: 637.3326, found: 637.3326.

### Methyl (S)-2-(((S)-2-azido-3-phenylpropanoyl)-L-leucyl-L-leucyl-D-leucyl)oxirane-2-carboxylate (C).

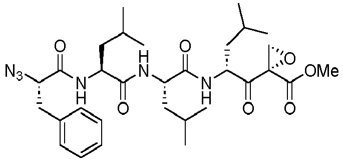

White solid (yield 97 mg, 0.158 mmol, 48.7 %):
¹H NMR (400 MHz, CDCl₃) δ 7.37 - 7.22 (m, 5H), 6.67 (d, *J=* 7.9 Hz, 1H), 6.55 (d, *J* = 6.4 Hz, 1H), 6.23 (d, *J* = 8.4 Hz, 1H), 4.64 (ddd, J = 11.0, 8.0, 3.2 Hz, 1H), 4.48 (ddd, *J* = 9.4, 8.6, 5.1 Hz, 1H), 4.35 (dd, *J* = 7.4, 4.2 Hz, 1H), 4.21 (dt, J = 9.4, 5.8 Hz, 1H), 3.80 (s, 3H), 3.40 (d, *J =* 5.9 Hz, 1H), 3.36 (d, *J =* 5.9 Hz, 1H), 3.31 (dd, *J=* 14.1, 4.2 Hz, 1H), 3.07 (dd, *J =* 14.1, 7.4 Hz, 1H), 1.81 - 1.69 (m, 2H), 1.68 - 1.52 (m, 3H), 1.52 - 1.34 (m, 4H), 0.97 (d, *J* = 6.6 Hz, 3H), 0.95 (d, *J =* 6.5 Hz, 3H), 0.92 (d, *J* = 6.6 Hz, 3H), 0.90 (d, *J* = 6.5 Hz, 3H), 0.89 (d, *J* = 6.4 Hz, 3H), 0.84 (d, *J* = 6.5 Hz, 3H) ppm;
¹³C NMR (101 MHz, CDCl₃) δ 202.11, 172.19, 171.68, 169.75, 166.01, 135.80, 129.68, 128.87, 127.52, 65.35, 59.16, 53.38, 52.91, 52.87, 51.37, 51.21, 40.51, 40.14, 38.77, 38.38, 25.32, 25.04, 24.85, 23.48, 23.15, 23.03, 21.95, 21.83, 21.25 ppm;
HRMS (ESI) *m*/*z* calcd for C₃₁H₄₈N₆O₇ [M+Na]+: 637.3326, found: 637.3326.

### Methyl (R)-2-(((S)-2-azido-3-phenylpropanoyl)-L-leucyl-L-leucyl-L-leucyl)oxirane-2-carboxylate (D).

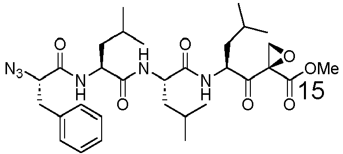

White solid (yield 57 mg, 0.093 mmol, 36.5 %):
¹H NMR (400 MHz, CDCl₃) δ 7.36 - 7.20 (m, 5H), 6.60 (d, *J* = 8.0 Hz, 1H), 6.42 (d, *J* = 7.9 Hz, 1H), 6.38 (d, *J* = 7.9 Hz, 1H), 4.67 (ddd, J = 10.5, 8.0, 2.7 Hz, 1H), 4.42 - 4.36 (m, 1H), 4.35 - 4.29 (m, 1H), 4.25 (dd, *J* = 7.6, 4.1 Hz, 1H), 3.81 (s, 3H), 3.43 (d, *J =* 5.9 Hz, 1H), 3.31 (d, *J* = 5.9 Hz, 1H), 3.31 (dd, *J* = 14.2, 4.0 Hz, 1H), 3.06 (dd, J = 14.1, 7.6 Hz, 1H), 1.73 - 1.57 (m, 4H), 1.57 - 1.47 (m, 2H), 1.47 - 1.30 (m, 3H), 0.98 - 0.92 (m, *J* = 6.3 Hz, 9H), 0.90 (d, *J* = 6.4 Hz, 3H), 0.86 (d, *J* = 6.6 Hz, 3H), 0.84 (d, *J* = 6.5 Hz, 3H) ppm;
¹³C NMR (101 MHz, CDCl₃) δ 201.70, 171.92, 171.65, 168.84, 165.86, 135.87, 129.68, 128.83, 127.47, 65.42, 59.03, 53.45, 52.54, 51.88, 51.73, 51.24, 40.76, 40.64, 39.22, 38.41, 25.26, 24.88, 24.64, 23.47, 22.98, 22.90, 22.28, 22.15, 21.25 ppm;
HRMS (ESI) *m*/*z* calcd for C₃₁H₄₈N₆O₇ [M+Na]⁺: 637.3326, found: 637.3326.

### Experimental procedure for the synthesis of the amides

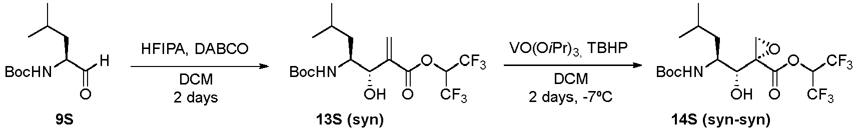

To a solution of **9S** (216 mg, 1 mmol, 1 equiv.) in DCM (0.4 mL, 0.4 mL/mmol), HFIPA (0.648 mL, 4 mmol, 4 equiv.) and DABCO (112.17 mg, 1 mmol, 1 equiv.) were added. The mixture was stirred (under N₂ atmosphere) for 2 days at room temperature. After the reaction completion (monitored by TLC), the mixture was diluted with DCM (78.4 mL, 3.5 mL/mmol) and quenched by adding 1 M HCl (40.3 mL, 1.8 mL/mmol). Then, it was extracted with DCM (2 x **78.4** mL, 2 x 3.5 mL/mmol) and organic layers were washed with brine (145.5 mL, 6.5 mL/mmol), dried over MgSO₄, and concentrated under vacuum. The crude material was purified by chromatography (silica gel, Hex:EtOAc:MeOH 9:1:0.2 to 7:3:0.2) to afford the pure compound.

### 1,1,1,3,3,3-hexafluoropropan-2-yl (3S,4S)-4-((tert-butoxycarbonyl)amino)-3-hydroxy-6-methyl-2-methyleneheptanoate (13S (syn)).

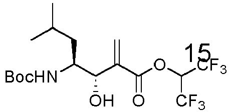

Catalyst β-ICD. Colourless oil:
¹H NMR (400 MHz, CDCl₃) (rotamers mixture) δ 6.51 (s, 1H), 6.20 (s, 1H), 5.91 - 5.78 (m, 1H), 4.65 (d, *J* = 9.0 Hz, 1H), 4.55 (s, 1H), 3.93 - 3.81 (m, 1H), 3.29 (s, 1H), 1.75 - 1.63 (m, 1H), 1.60 - 1.49 (m, 1H), 1.46 - 1.39 (m, 1H), 1.36 (s, 9H), 0.94 (d, *J* = 6.6 Hz, 3H), 0.92 (d, *J* = 6.5 Hz, 3H) ppm;
¹³C NMR (101 MHz, CDCl₃) δ 162.46, 156.64, 138.91, 129.46, 120.61 (q, *J* = 281.8 Hz), 79.71, 72.38, 66.80 (hept, *J* = 33.8 Hz), 51.87, 40.93, 28.27, 25.18, 23.00, 22.31 ppm;
¹⁹F NMR (377 MHz, CDCl₃) δ -73.05 - -73.29 (m) ppm;
HRMS (ESI) *m*/*z* calcd for C₁₇H₂₅F₆NO₅ [M+H]⁺: 438.1716, found: 438.1715.

To a -7 °C cold solution of alkene (13) (1 equiv.) in DCM (12.5 mL/mmol), VO(OiPr)₃ (0.2 equiv.) and TBHP (5 equiv.) were added. The reaction mixture was stirred for 2 days and was monitored by TLC. Then, saturated aqueous solution of Na₂S₂O₃ (12.5 mL/mmol) was added and the mixture was stirred for 15-20 min. Then, it was extracted with DCM (3 x 12.5 mL/mmol), washed with saturated aqueous solution of Na₂S₂O₃ (12.5 mL/mmol), dried over MgSO₄, filtered, and concentrated under vacuum. The crude material was purified by silica gel chromatography.

### 1,1,1,3,3,3-hexafluoropropan-2-yl (S)-2-((1R,2S)-2-((tert-butoxycarbonyl)amino)-1-hydroxy-4-methylpentyl)oxirane-2-carboxylate (14S (syn-syn)).

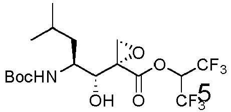

Hex/EtOAc 9:1 to 7:3. Colourless oil (yield 3.661 mg, 8.07 mmol, 86.5 %):
¹H NMR (400 MHz, CDCl₃) δ 5.87 - 5.75 (m, 1H), 4.52 (d, *J* = 9.3 Hz, 1H), 4.22 (s, 1H), 4.21 - 4.10 (m, 1H), 3.25 (d, *J =* 5.8 Hz, 1H), 3.20 (d, *J =* 5.9 Hz, 1H), 2.18 (s, 1H), 1.77 - 1.67 (m, 1H), 1.60 - 1.51 (m, 1H), 1.50 - 1.43 (m, 1H), 1.39 (s, 9H), 0.97 (d, *J* = 6.6 Hz, 3H), 0.93 (d, *J* = 6.5 Hz, 3H) ppm;
¹³C NMR (101 MHz, CDCl₃) δ 166.37, 156.15, 120.32 (q, *J* = 281.6 Hz), 79.71, 67.23 (hept, *J* = 35.4 Hz), 56.39, 49.20, 48.11, 41.34, 28.35, 24.99, 22.91, 22.27 ppm.
¹⁹F NMR (377 MHz, CDCl₃) δ -72.90 - -73.11 (m) ppm.

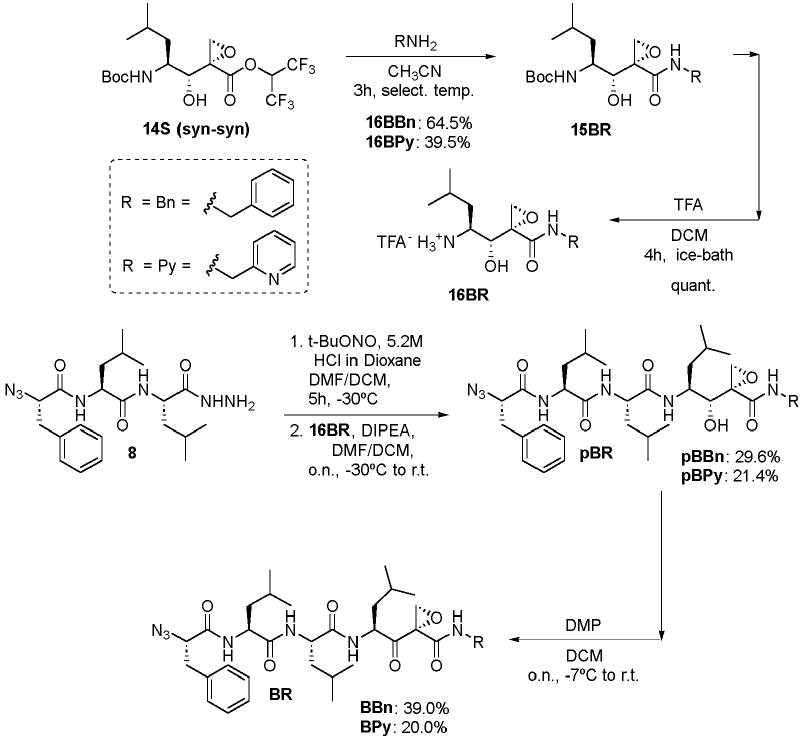

To a cold solution of **14S (syn-syn)** (1 equiv.) in CH₃CN (2 mL/mmol), benzylamine or picolylamine (1.2 equiv.) was added and the solution was stirred until TLC showed starting material consumption (3-4 h). The reaction mixture was concentrated under vacuum and the amide was separated from the excess of amine on a pad of silica gel. The crude material was purified by silica gel chromatography.

### tert-Butyl ((1R,2S)-1-((S)-2-(benzylcarbamoyl)oxiran-2-yl)-1-hydroxy-4-methylpentan-2-yl)carbamate (15BBn).

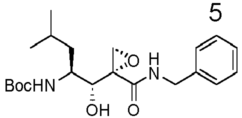

Benzylamine. Hex:EtOAc 7:3 to 6:4. Colourless oil: (yield 415 mg, 1.06 mmol, 64.5 %):
¹H NMR (400 MHz, CDCl₃) δ 7.36 - 7.26 (m, 3H), 7.24 - 7.19 (m, 2H), 6.97 (s, *J* = 9.6 Hz, 1H), 4.66 (d, *J* = 9.7 Hz, 1H), 4.58 (dd, *J* = 14.7, 6.9 Hz, 1H), 4.35 (d, *J* = 6.8 Hz, 1H), 4.20 (dd, *J* = 14.7, 4.7 Hz, 1H), 4.10 - 3.98 (m, 1H), 3.52 (d, *J* = 5.1 Hz, 1H), 3.04 (d, *J* = 5.0 Hz, 1H), 2.85 (d, *J* = 5.1 Hz, 1H), 1.67 - 1.58 (m, 1H), 1.58 - 1.50 (m, 1H), 1.44 (s, 9H), 1.36 - 1.27 (m, 1H), 0.92 (d, *J* = 6.6 Hz, 3H), 0.91 (d, *J =* 6.5 Hz, 3H) ppm;
¹³C NMR (101 MHz, CDCl₃) δ 170.36, 156.20, 137.45, 128.95, 127.86, 79.49, 74.81, 57.14, 53.01, 50.57, 43.07, 42.03, 28.56, 24.84, 23.26, 22.14 ppm;
HRMS (ESI) *m*/*z* calcd for C₂₁H₃₂N₂O₅ [M+H]⁺: 393.2389, found: 393.2394.

### tert-Butyl ((1R,2S)-1-hydroxy-4-methyl-1-((S)-2-((pyridin-2-ylmethyl)carbamoyl)oxiran-2-yl)pentan-2-yl)carbamate (15BPy).

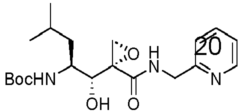

Picolylamine. Hex:EtOAc:MeOH 3:7:0.1 to 0:10:0.1. Yellowish oil: (yield 407 mg, 1.03 mmol, 39.5 %):
¹H NMR (400 MHz, CDCl₃) δ 8.53 (ddd, *J* = 4.9, 1.6, 0.9 Hz, 1H), 7.65 (td, *J* = 7.7, 1.8 Hz, 1H), 7.61 (s, *J* = 42.8 Hz, 1H), 7.24 - 7.15 (m, 1H), 4.70 - 4.62 (m, 1H), 4.59 (d, *J =* 5.7 Hz, 1H), 4.45 (d, *J* = 4.3 Hz, 1H), 4.41 (d, *J =* 4.6 Hz, 1H), 4.10 (tdd, *J* = 9.9, 4.9, 2.6 Hz, 1H), 3.53 (d, *J=* 4.2 Hz, 1H), 3.07 (d, *J =* 5.2 Hz, 1H), 2.96 (d, *J* = 5.2 Hz, 1H), 1.65 - 1.59 (m, 1H), 1.59 - 1.50 (m, 1H), 1.37 (s, 2H), 0.92 (d, *J* = 6.6 Hz, 1H), 0.91 (d, *J* = 6.5 Hz, 1H) ppm;
¹³C NMR (101 MHz, CDCl₃) δ 170.86, 156.03, 155.74, 149.44, 136.89, 122.64, 121.91, 79.30, 75.05, 56.94, 53.25, 50.68, 43.90, 41.97, 28.49, 24.86, 23.29, 22.19 ppm;
HRMS (ESI) *m*/*z* calcd for C₂₀H₃₁N₃O₅ [M+H]⁺: 394.2342, found: 394.2344.

The resulting epoxy amides **(15BR)** were submitted to deprotection **(16BR),** coupling **(pBR)** and oxidation **(BR)** steps, following the same experimental procedures as described above for the carboxylate analogues.

### (S)-2-((1R,2S)-2-((S)-2-((S)-2-((S)-2-azido-3-phenylpropanamido)-4-methylpentanamido)-4-methylpentanamido)-1-hydroxy-4-methylpentyl)-N-benzyloxirane-2-carboxamide (pBBn).

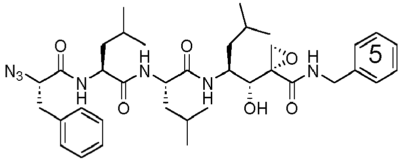

Hex:EtOAc:MeOH 1:1:0.1 to 4:6:0.1. White solid (yield 203 mg, 0.29 mmol, 29.6 %):
¹H NMR (400 MHz, CDCl₃) δ 7.34 - 7.19 (m, 10H), 7.02 (t, *J* = 5.9 Hz, 1H), 6.78 (d, *J =* 8.0 Hz, 1H), 6.72 (d, *J* = 8.2 Hz, 1H), 6.57 (d, *J* = 9.4 Hz, 1H), 4.49 (dd, *J* = 14.8, 6.5 Hz, 1H), 4.44 - 4.33 (m, 3H), 4.31 - 4.20 (m, 2H), 3.65 (dd, *J* = 6.9, 2.6 Hz, 1H), 3.27 (dd, *J =* 14.1, 4.2 Hz, 1H), 3.04 (dd, *J* = 13.9, 7.3 Hz, 1H), 3.02 (d, *J* = 5.2 Hz, 1H), 2.81 (d, *J* = 5.1 Hz, 1H), 2.05 (s, 1H), 1.71 - 1.47 (m, 6H), 1.38 (ddt, *J* = 13.7, 9.2, 5.3 Hz, 3H), 0.97 - 0.84 (m, 15H), 0.84 (d, *J* = 6.5 Hz, 3H) ppm;
¹³C NMR (101 MHz, CDCl₃) δ 172.05, 171.68, 169.85, 168.96, 137.46, 135.93, 129.63, 128.93, 128.83, 128.77, 127.85, 127.78, 127.73, 127.39, 73.77, 65.26, 57.72, 52.41, 52.37, 51.88, 49.52, 43.02, 41.37, 41.01, 40.88, 38.33, 24.91, 24.75, 24.65, 23.34, 23.13, 22.92, 22.26, 22.08, 21.99 ppm;
HRMS (ESI) *m*/*z* calcd for C₃₇H₅₃N₇O₆ [M+H]⁺: 692.4136, found: 692.4138.

### (S)-2-((1R,2S)-2-((S)-2-((S)-2-((S)-2-azido-3-phenylpropanamido)-4-methylpentanamido)-4-methylpentanamido)-1-hydroxy-4-methylpentyl)-N-(pyridin-2-ylmethyl)oxirane-2-carboxamide (pBPy).

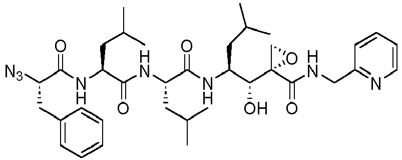

Hex:EtOAc:MeOH 2:8:0.1 to 0:10:0.1. Colourless oil (yield 146 mg, 0.21 mmol, 21.4 %):
¹H NMR (400 MHz, CDCl₃) δ 8.52 (dd, *J =* 4.8, 0.6 Hz, 1H), 7.65 (td, *J* = 7.7, 1.8 Hz, 1H), 7.61 (t, *J =* 5.4 Hz, 1H), 7.33 - 7.17 (m, 7H), 6.68 (d, *J* = 7.7 Hz, 1H), 6.66 (d, *J* = 8.9 Hz, 1H), 6.47 (d, *J* = 8.0 Hz, 1H), 4.57 (dd, *J* = 16.2, 5.7 Hz, 1H), 4.47 (dd, *J* = 16.2, 5.1 Hz, 1H), 4.39 - 4.28 (m, 3H), 4.25 (dd, *J* = 7.7, 4.2 Hz, 1H), 3.70 (d, *J* = 2.4 Hz, 1H), 3.30 (dd, *J* = 14.1, 4.2 Hz, 1H), 3.05 (dd, *J* = 14.5, 7.3 Hz, 1H), 3.05 (d, *J* = 5.1 Hz, 1H), 2.92 (d, *J* = 5.1 Hz, 1H), 1.67 - 1.46 (m, 6H), 1.46 - 1.32 (m, 3H), 1.25 (s, 1H), 0.91 (d, *J* = 6.4 Hz, 3H), 0.90 - 0.85 (m, 12H), 0.84 (d, *J* = 6.6 Hz, 3H) ppm;
¹³C NMR (101 MHz, CDCl₃) δ 171.95, 171.69, 170.11, 168.80, 156.08, 149.27, 137.01, 135.99, 129.62, 128.74, 127.34, 122.67, 122.16, 73.44, 65.24, 58.01, 52.36, 52.10, 51.79, 49.71, 43.86, 41.15, 40.95, 38.31, 24.86, 24.78, 24.62, 23.34, 23.07, 22.93, 22.22, 22.15, 22.02 ppm;
HRMS (ESI) *m*/*z* calcd for C₃₆H₅₂N₈O₆ [M+H]⁺: 693.4088, found: 693.4089.

### (S)-2-(((S)-2-azido-3-phenylpropanoyl)-L-leucyl-L-leucyl-L-leucyl)-N-benzyloxirane-2-carboxamide (BBn).

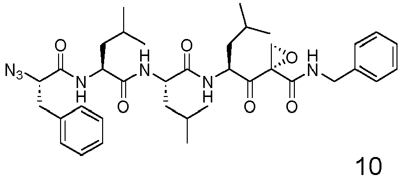

Hex:EtOAc:MeOH 1:1:0.1 to 4:6:0.1. Colourless oil: (yield 66 mg, 0.096 mmol, 39.0 %):
¹H NMR (400 MHz, CDCl₃) δ 7.37 - 7.20 (m, 10H), 7.08 (t, *J* = 5.6 Hz, 1H), 6.73 (d, *J =* 7.2 Hz, 1H), 6.63 (d, *J=* 7.9 Hz, 1H), 6.47 (d, *J=* 8.1 Hz, 1H), 4.98 (ddd, *J* = 10.3, 7.2, 3.4 Hz, 1H), 4.52 - 4.43 (m, 2H), 4.43 - 4.36 (m, 1H), 4.36 - 4.29 (m, 1H), 4.26 (dd, *J* = 7.6, 4.2 Hz, 1H), 3.30 (dd, *J* = 14.1, 4.2 Hz, 1H), 3.19 (d, *J* = 6.1 Hz, 1H), 3.11 (d, *J* = 6.1 Hz, 1H), 3.06 (dd, *J* = 14.1, 7.6 Hz, 1H), 1.68 - 1.31 (m, 9H), 0.97 - 0.87 (m, 12H), 0.86 (d, *J* = 6.6 Hz, 3H), 0.84 (d, *J* = 6.5 Hz, 3H) ppm;
¹³C NMR (101 MHz, CDCl₃) δ 201.82, 171.77, 171.73, 168.96, 165.19, 137.41, 135.90, 129.67, 128.96, 127.94, 127.91, 127.45, 65.38, 59.16, 55.17, 52.96, 51.93, 51.67, 43.59, 40.82, 40.40, 39.08, 38.37, 25.11, 24.89, 24.66, 23.51, 22.99, 22.96, 22.17, 22.10, 21.39 ppm;
HRMS (ESI) *m*/*z* calcd for C₃₇H₅₁N₇O₆ [M+Na]⁺: 712.3802, found: 712.3799.

### (S)-2-(((S)-2-azido-3-phenylpropanoyl)-L-leucyl-L-leucyl-L-leucyl)-N-(pyridin-2-ylmethyl)oxirane-2-carboxamide (BPy).

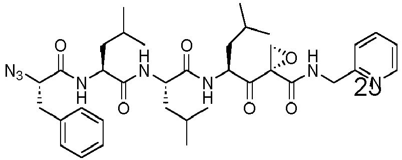

Hex:EtOAc:MeOH 2:8:0.1 to 0:10:0.1. Colourless oil: (yield 24 mg, 0.035 mmol, 20.0 %):
¹H NMR (400 MHz, CDCl₃) δ 8.54 (ddd, *J* = 4.9, 1.6, 0.9 Hz, 1H), 7.90 (t, *J=* 5.4 Hz, 1H), 7.69 (td, *J* = 7.7, 1.8 Hz, 1H), 7.50 (d, *J =* 7.7 Hz, 1H), 7.33 - 7.19 (m, 7H), 6.60 (d, *J* = 8.0 Hz, 1H), 6.53 (d, *J* = 8.0 Hz, 1H), 4.87 - 4.76 (m, 1H), 4.64 (dd, *J* = 16.4, 5.6 Hz, 1H), 4.57 (dd, *J* = 16.3, 5.5 Hz, 1H), 4.43 - 4.35 (m, 1H), 4.35 - 4.29 (m, 1H), 4.25 (dd, *J* = 7.6, 4.2 Hz, 1H), 3.30 (dd, *J=* 14.1, 4.1 Hz, 1H), 3.17 (d, *J=* 6.0 Hz, 1H), 3.13 (d, *J* = 6.0 Hz, 1H), 3.05 (dd, *J =* 14.1, 7.6 Hz, 1H), 1.65 - 1.50 (m, 6H), 1.45 - 1.30 (m, 3H), 0.91 (d, *J =* 6.4 Hz, 3H), 0.90 - 0.80 (m, 15H) ppm;
¹³C NMR (101 MHz, CDCl₃) δ 201.40, 172.00, 171.96, 168.82, 166.09, 156.18, 149.12, 137.28, 135.90, 129.68, 128.81, 127.45, 122.83, 122.33, 65.43, 59.48, 55.69, 52.87, 51.92, 51.82, 44.27, 40.88, 40.74, 38.81, 38.40, 25.10, 24.89, 24.61, 23.49, 23.06, 23.01, 22.06, 21.99, 21.43 ppm;
HRMS (ESI) *m*/*z* calcd for C₃₆H₅₀N₈O₆ [M+H]⁺: 691.3936, found: 691.3932.

### Experimental part of the biological assays

Lysates of cells were prepared by treating cell pellets with 4 volumes of lysis buffer containing 50 mM Tris pH 7.5, 2 mM DTT, 5 mM MgCl₂, 10% glycerol, 2 mM ATP and 0.05% digitonin for 15-60 min. Protein concentration was determined using Qubit^{®} protein assay kit. All cell lysate labelling experiments were performed in assay buffer containing 50 mM Tris pH 7.5, 2 mM DTT, 5 mM MgCl₂, 10% glycerol, 2 mM ATP. Cell lysate labelling and competition experiments were performed at 37 °C. Prior to fractionation on 12.5% SDS-PAGE (TRIS/glycine), samples were boiled for 3 min in a reducing gel loading buffer. The 7.5x10 cm (L x W) gels were run for 15 min at 80V followed by 120 min at 130V. In-gel detection of (residual) proteasome activity was performed in the wet gel slabs directly on a ChemiDoc^{®} MP System using Cy2 setting to detect BODIPY(FL)-LU-112, BODIPY(FL)-epoxomycin and BODIPY(FL)-NC-001, Cy3 settings to detect BODIPY(TMR)-NC-005-VS and BODIPY(TMR)-NC-005-VS and BODIPY(TMR)-epoxomycin and BODIPY(FL)-NC-001 and Cy5 setting to detect Cy5-NC-001. When the probes were used as a mixture the following concentrations were used: 100 nM Cy5-NC-001, 30 nM BODIPY(FL)-LU-112, 100 nM BODIPY(TMR)-NC-005-VS, as premixed 10x concentration cocktail in DMSO which was incubated with cell lysate for 60 min.

### Competition experiments in cell lysate

Cell lysates (diluted to 10-15micrograms total protein in 9 microliter buffer) were exposed to the inhibitors (10x stock in DMSO) at indicated concentrations for 1h at 37 °C, followed by addition of probe cocktail (10x stock, 1.1 microliters) and incubation for 1h, finally SDS-PAGE as described above.

Inhibitors **A-D** were tested against proteasome at Leiden University through competitive assays with activity-based probes (ABPs) specific for each catalytic subunit (b1, b2, b5) of both constitutive and immuno-proteasomes developed at Leiden University (G. Bruin, B. T. Xin, M. Kraus, M. Stelt, G.A. van der Marel, A. F. Kisselev, C. Driessen, B. I. Florea, H. S. Overkleeft Angew. Chem. Int. Ed. 2016, 55, 4199 -4203). Compounds A, B, C and D were tested against proteasome from Raji cells.

The four isomeric epoxyketones A, B, C and D showed in vitro activity against constitutive and immunoproteasome subunits (Figure 1). Inhibitors B and D were the most active ones. Interestingly, compound B having an opposite stereochemistry of the epoxide to epoxomycin gave an applC50 of 15nM as compared to compound D having the same stereochemistry as epoxomycin. Inhibitor B also discriminates between b5c and b5i.

Amides **BBn** and **Bpy,** derived from isomer B, were assayed as inhibitors of three catalytic subunits of both constitutive and immunoproteasome. Competitive experiments of the inhibitors at nanomolar and micromolar level with selective ABPs were performed for both **BBn, Bpy,** and for comparison compound B was also assayed (Figure 2).

Amides **BBn** and **Bpy** were more active than **B** for all subunits. Remarkably, apparent *in vitro* IC50 of b2c (and b2i) for both amides were less than one order of magnitude than inhibitor **B, Bpy** was more active than **BBn,** and both **BBn** and **Bpy** were more active than **B.** These results suggest that adding a substituent to fill in the S1' site of the active center of the subunits b2c, b2i, b5c and b5i has a high positive effect in the activity of peptidyl epoxy ketones inhibitors (Figure 3, and Figure 4).

For the *in vivo* experiments, apparent IC50 values for **BBn** and **Bpy** were also calculated (Figure 5).

## Claims

1. A compound of formula **I**: 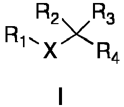 or a pharmaceutically acceptable salt thereof, wherein:
X is C=O or C=S or CH-OCOCH₃;
R₁ is a peptidic group binding to a proteolytic site of a proteasome, wherein said peptidic group is bound to X, comprises at least two amino acids, and X takes the place of the carbonyl group of the C-terminal amino acid; and optionally a second group enhancing delivery, wherein said second group is bound to the N-terminus of said peptidic group;
when R₂ is CH₂ or CH-alkyl, R₃ is O, whereby R₂ and R₃ forming an epoxide ring; or
when R₂ is CH=O, CH₂-I, CH₂-Br, CH₂-Cl, CH₂-OPO(OH)₂, CH₂-p-toluene sulfonyloxy (CH₂-OTs), CH₂-methane sulfonyloxy (CH₂-Oms) or CO- N-oxy-succinimide (CO-NHS), R₃ is H or methyl or CF₃;
R₄ is C(O)-YR₅ or C(S)-YR₅, or CH₂YR₅;
Y is NH, C, O or S; and
R₅ is a group occupying the new site binding, particularly an alkyl, aryl or heteroaryl group to establish pi-pi interactions with Tyr-169, that group may be substituted, allowing formation of hydrogen bonds with Thr-21 (constitutive proteasome) / Ser-21 (immunoproteasome), an aromatic ring present in an amino acid containing an aromatic group or R₅ is a peptidic group.

2. The compound according to claim 1 wherein R₂ and R₃ forming an epoxide ring.

3. The compound according to claim 1 or 2 wherein Y is O or NH.

4. The compound according to any one of the preceding claims wherein R₅ is a six membered aryl group or a six membered heteroaryl group.

5. The compound according to claim 4 wherein R₄ is a CH₂-Aryl group or a CH₂-heteroarylgroup.

6. The compound according to claim 4 or 5 wherein the aryl or heteroaryl group is selected from phenyl, 2-pyridine, 3-pyridine or 4-pyridine.

7. The compound according to any one of claims 1 to 3 wherein Y is O or NH and R₅ is a C1-C4 alkyl group, in particular, a methyl group.

8. The compound according to any one of the preceding claims wherein the aromatic amino acid is Tyr.

9. A pharmaceutical composition, which comprises a compound of formula **I** according to any one of claims 1 to 8 or a pharmaceutically acceptable salt thereof and one or more pharmaceutically acceptable excipients.

10. A compound of formula **I** according to any of claims 1 to 8 or a pharmaceutical salt thereof for use as a medicament.

11. A compound of formula **I** according to any of claims 1 to 8 or a pharmaceutical salt thereof for use as a proteasome inhibitor

12. The compound of formula I for the use according to claim 11, wherein the inhibitor interacts with Tyr-169, and Thr-21 (constitutive proteasome) / Ser-21 (immunoproteasome).

13. A compound of formula **I** or a pharmaceutical salt thereof according to any one of claims 1 to 8 or the pharmaceutical composition according to claim 9 for use in the treatment of a disease selected from cancer, an autoimmune disease, muscular dystrophy, emphysema, cachexia accompanying cancer or AIDS.

14. The compound of formula **I** or a pharmaceutical salt thereof or the pharmaceutical composition for the use according to claim 13, wherein cancer is lymphoid malignancy, non-Hodgkin lymphoma or Waldenstrom macroglobulinaemia.

15. The compound of formula **I** or a pharmaceutical salt thereof or the pharmaceutical composition for the use according to claim 13, wherein autoimmune disease is rheumatoid arthritis, systemic lupus erythematosus, Sjorgen's syndrome or scleroderma.
